# EUROPEAN PATENT APPLICATION

(11) **EP 2 770 054 A1**
(43) Date of publication of application: **27.08.2014**
(21) Application number: 12842392.8
(22) Date of filing: 18.10.2012
(51) Int. Cl.: C12N 15/09, A61K 48/00, C12N 15/113, C12N 15/115

(54) **ACTIVATION OF FUNCTIONAL NUCLEIC ACID BY SPECIFIC MODIFICATION**

(30) Priority: 19.10.2011 JP 2011230090
(71) Applicant: Riken, Wako-shi, Saitama 351-0198 (JP); TAGCyx Biotechnologies, Yokohama-shi Kanagawa 230-0045 (JP)
(72) Inventor: HIRAO, Ichiro, Tokyo 2010004 (JP); HIRAO, Michiko, Tokyo 2010004 (JP); LIU, Shuang, Kanagawa 2300045 (JP); NOZAWA, Iwao, Kanagawa 2300045 (JP)
(74) Representative: Denison, Christopher Marcus
(86) International application number: PCT/JP2012/076911
(87) International publication number: WO 2013/058306

(57) **Abstract**

This invention is intended to enhance and improve the resistance of a single- or double-stranded nucleic acid fragment comprising a base sequence of a functional nucleic acid to degradation by nucleolytic enzymes in a simple and cost-effective manner. The single- or double-stranded nucleic acid fragment comprises, ligated to at least one 3' end thereof, a hairpin-shaped DNA comprising: (A) a nucleic acid region consisting of 2 to 5 arbitrary nucleotides; (B) a nucleic acid region consisting of a "gna" or "gnna" base sequence, wherein each "n" represents "g", "t", "a", or "c", a base analogue, or a modified base; and (C) a nucleic acid region consisting of a base sequence complementary to the nucleic acid region (A), sequentially ligated from the 5' end toward the 3' end, wherein at least one of two 3' terminal nucleotides from at least one 3' end of the single-stranded nucleic acid fragment or the double-stranded nucleic acid fragment is modified.

SEQUENCE LISTING

<110>  RIKEN
       TagCyx Biotechnologies

<120>  Activation of functional nucleic acids by specific modifications

<130>  PH-5383-PCT

<150>  JP 2011-230090
<151>  2011-10-19

<160>  7

<170>  PatentIn version 3.5

<210>  1
<211>  25
<212>  RNA
<213>  Artificial

<220>
<223>  Photinus pyralis luciferase siRNA

<400>  1
aaggagccuu caggauuaca agauu                                          25


<210>  2
<211>  25
<212>  RNA
<213>  Artificial

<220>
<223>  Photinus pyralis luciferase siRNA

<400>  2
aaucuuguaa uccugaaggc uccuu                                          25


<210>  3
<211>  34
<212>  DNA
<213>  Artificial

<220>
<221>  RNA
<222>  (1)..(25)
<223>  Photinus pyralis luciferase siRNA/DNA chimera

<400>  3
aaggagccuu caggauuaca agauucgcga agcg                                34


<210>  4
<211>  34
<212>  DNA
<213>  Artificial

<220>
<221>  RNA
<222>  (1)..(25)
<223>  Photinus pyralis luciferase siRNA/DNA chimera

<400>  4
aaucuuguaa uccugaaggc uccuucgcga agcg                                    34


<210>  5
<211>  43
<212>  DNA
<213>  Artificial

<220>
<221>  RNA
<222>  (10)..(34)
<223>  Photinus pyralis luciferase siRNA/DNA chimera

<400>  5
cgcgaagcga aggagccuuc aggauuacaa gauucgcgaa gcg                          43


<210>  6
<211>  7
<212>  DNA
<213>  Artificial

<220>
<223>  mini-hairpin

<400>  6
gcgaagc                                                                   7


<210>  7
<211>  10
<212>  DNA
<213>  Artificial

<220>
<223>  mini-hairpin

<400>  7
cgcgaaagcg                                                               10

## Description

### Technical Field

The present invention relates to a method for enhancing the resistance of a nucleic acid fragment to degradation by a nucleolytic enzyme and a nucleic acid having such properties.

### Background Art

Functional nucleic acids, such as siRNAs, nucleic acid aptamers, and decoy nucleic acids, have drawn attention as pharmaceuticals or diagnostic agents in recent years, and research on and development of a variety of nucleic acid pharmaceuticals and the like are in progress with the goal of establishing medical applications for the same all over the world.

However, nucleic acids are generally problematic in that they are likely to be degraded by nucleolytic enzymes, such as nucleases*, in vivo.* In particular, siRNAs or RNA aptamers that have recently drawn attention as nucleic acid pharmaceuticals because of the applicability and effects thereof are composed of RNAs, which are very unstable *in vivo.* Accordingly, *in vivo* stability of nucleic acid is essential for the efficient and continuous exertion of the pharmacological effects of nucleic acid pharmaceuticals. In addition, much still remains unknown about activation mechanisms, including stabilization by modified nucleic acids, underlying the intracellular physiological activity of the nucleic acid pharmaceuticals.

Many methods aimed at nucleic acid stabilization and stabilized nucleic acids have heretofore been reported. An example is a method for stabilization of decoy nucleic acids using a dumbbell shape (Patent Literatures 1 to 3). Formation of a dumbbell-shaped nucleic acid is a method in which both ends of a double-stranded nucleic acid fragment are ligated to each other with a loop structure, such as a linker nucleic acid, to form a closed circle, so that the double-stranded nucleic acid fragment acquires resistance to degradation by a nucleolytic enzyme. This method, however, disadvantageously necessitates a process of cyclization of a linear nucleic acid fragment, which is complicated and high cost.

Use of artificially constructed artificial nucleic acids that are not be degraded by nucleases is also taken into consideration. However, the application of artificial nucleic acids that are not degraded *in vivo* for pharmaceutical products remains problematic from the viewpoint of safety, such as with regard to side effects. Although the modification of nucleic acids for stabilization enhances their stability against nucleases, the physiological activity of these modified nucleic acids is hardly enhanced.

Accordingly, development of nucleic acid pharmaceuticals that are composed to as a great extent as possible of naturally occurring nucleic acids, highly resistant to degradation by nucleolytic enzymes, stably maintained *in vivo,* and easily prepared in a simple and cost-effective manner has been awaited.

### Citation List

### Patent Literature

Patent Literature 1: WO 2003/091432
Patent Literature 2: WO 2005/014810
Patent Literature 3: US 2003/040613

### Summary of Invention

### Technical Problem

It is an object of the present invention to provide a method for easily enhancing the resistance of a double-stranded nucleic acid fragment or a single-stranded nucleic acid fragment forming a higher-order structure via intramolecular folding to degradation by a nucleolytic enzyme in a cost-effective manner and a nucleic acid molecule obtained by such method.

The present invention provides a nucleic acid molecule capable of enhancing the stability of a comprised functional nucleic acid *in* vivo and allowing the same to continuously exert its pharmacological effects.

### Solution to Problem

The present inventors have conducted concentrated studies in order to attain the above object. As a result, they discovered that: ligation of a hairpin-shaped DNA consisting of a specific sequence to at least one 3' end of a single- or double-stranded nucleic acid fragment and modification of at least one of two 3' terminal nucleotides from at least one 3' end of the nucleic acid fragment may enhance resistance of the nucleic acid fragment to degradation by a nucleolytic enzyme, compared with mere ligation of the hairpin-shaped DNA to the nucleic acid fragment, even if the fragment is not in a closed circular form; and the resulting nucleic acid fragment may be not only stabilized but may maintain and enhance its biological activity, when comprising a functional nucleic acid.

The above-mentioned "hairpin-shaped DNA consisting of a specific sequence" is a nucleic acid molecule consisting of specific base sequences having 7 to 14 bases and forming a hairpin structure via intramolecular folding. It has been known that a hairpin-shaped DNA is ligated to a single-stranded nucleic acid fragment, such as mRNA or primer DNA, which would not form a higher-order structure via intramolecular folding, so that the hairpin-shaped DNA would be capable of imparting the end of the single-stranded nucleic acid fragment with resistance to degradation by a nucleolytic enzyme (Hirao I. et al., 1990, Nucleic Acid Symp. Ser., 22, 75-76; Hirao I. et al., 1993, FEBS Lett., 321, 169-172; Khan I. M. & Coulson J. M., 1993, Nucleic Acids Res., 21, 2967-2958; Yoshizawa S. et al., 1994, Nucleic Acids Res. 22, 2217-2221; Hirao I. et al., 1994, Nucleic Acids Res. 22, 576-582; Mestre B. et al., 1995, Bioconjug. Chem., 6, 466-472; Yoshizawa S. et al., 1997, Biochemistry 36, 4761-4767; Jolles B. et al., 1997, Nucleic Acids Res. 25, 4608-4613).

When the hairpin-shaped DNA consisting of a specific sequence was ligated to a double-stranded nucleic acid fragment or a single-stranded nucleic acid fragment forming a higher-order structure via intramolecular folding that contains functional nucleic acids, it was not known that similar nucleolytic enzyme resistance effects could be attained or functions of functional nucleic acids could be maintained or not. In addition, a dumbbell-shaped structure for nucleic acid stabilization is a closed circular structure as described above, and it has not been even deduced in the art that a dumbbell-like structure having a non-ligated region (i.e., a dumbbell-shaped structure having 1 or 2 nicks) has resistance to degradation by a nucleolytic enzyme. It has heretofore been known that chemical modification (e.g., 2'-O-methyl substitution) of a 2'-hydroxy group in a ribose sugar backbone imparts nucleolytic enzyme resistance to a functional nucleic acid molecule, particularly, antisense RNA or siRNA. However, it has never been expected that such chemical modification can also maintain or enhance the biological activity (e.g., antisense effect or RNAi activity) of the functional nucleic acid molecule.

The present invention has been completed based on the above result and provides (1) to (17) below.

(1) A nucleic acid molecule comprising: a hairpin-shaped DNA comprising nucleic acid regions (A) to (C) below sequentially ligated from the 5' end toward the 3' end:
   (A) a first nucleic acid region consisting of 2 to 5 arbitrary nucleotides;
   (B) a second nucleic acid region consisting of a "gna" or "gnna" base sequence, wherein each "n" independently represents "g", "t", "a", or "c", a base analogue, or a modified base; and
   (C) a third nucleic acid region consisting of a base sequence complementary to the first nucleic acid region,
   wherein the first nucleic acid region and the third nucleic acid region form a stem moiety by base pairing with each other and the second nucleic acid region forms a loop moiety, and a nucleic acid fragment represented (i) or (ii) below.
   (i) a double-stranded nucleic acid fragment made by complete or partial base pairing, wherein at least one of two 3' terminal nucleotides from at least one 3' end is modified; or
   (ii) a single-stranded nucleic acid fragment having at least one stem structure and at least one loop structure, wherein at least one of two 3'terminal nucleotides is modified, wherein at least one 3' end of the nucleic acid fragment is ligated to the hairpin-shaped DNA.
(2) The nucleic acid molecule according to (1), wherein the nucleic acid fragment is the double-stranded nucleic acid fragment (i) of (1), wherein at least one hairpin-shaped DNA-ligated nucleic acid strand of the nucleic acid fragment is modified.
(3) The nucleic acid molecule according to (1) or (2), wherein the first nucleic acid region consists of "g" or "c" base.
(4) The nucleic acid molecule according to any of (1) to (3), wherein the modified nucleotide in the nucleic acid fragment is composed of RNA.
(5) The nucleic acid molecule according to (4), wherein the modification is substitution of a 2'-hydroxy group in ribose.
(6) The nucleic acid molecule according to (5), wherein the hydroxy group is substituted by a methoxy group, an ethoxy group, a propoxy group, or a butoxy group.
(7) The nucleic acid molecule according to any of (1) to (6), wherein the nucleic acid fragment comprises a functional nucleic acid.
(8) The nucleic acid molecule according to (7), wherein the nucleic acid fragment is the double-stranded nucleic acid fragment (i) of (1), wherein the functional nucleic acid is an siRNA, a mature double-stranded miRNA, or a target molecule-binding nucleic acid fragment.
(9) The nucleic acid molecule according to (7), wherein the nucleic acid fragment is the single-stranded nucleic acid fragment (ii) of (1), wherein the functional nucleic acid is an shRNA, a single-stranded miRNA precursor, a nucleic acid aptamer, a ribozyme (including deoxyribozyme), a molecular beacon, a riboswitch, a U1 adaptor, or a target molecule-binding nucleic acid fragment.
(10) A pharmaceutical composition comprising, as an active ingredient, the nucleic acid molecule according to any of (1) to (9).
(11) The pharmaceutical composition according to (10), which comprises a pharmaceutically acceptable carrier.
(12) A method for producing a nucleic acid molecule with enhanced resistance to a nucleolytic enzyme, comprising:
   a modification step of modifying
      (i) at least one of two 3' terminal nucleotides from at least one 3' end of a double-stranded nucleic acid fragment made by complete or partial base pairing, or
      (ii) at least one of two 3' terminal nucleotides in a single-stranded nucleic acid fragment having at least one stem structure and at least one loop structure; and
   a ligation step of ligating, to at least one 3' end of the nucleic acid fragment,
   a hairpin-shaped DNA comprising the nucleic acid regions (A) to (C) below sequentially ligated from the 5' end toward the 3' end:
      (A) a first nucleic acid region consisting of 2 to 5 arbitrary nucleotides;
      (B) a second nucleic acid region consisting of a "gna" or "gnna" base sequence; wherein each "n" independently represents "g", "t", "a", or "c", a base analogue, or a modified base; and
      (C) a third nucleic acid region consisting of a base sequence complementary to the first nucleic acid region,
      wherein the first nucleic acid region and the third nucleic acid region form a stem moiety by base pairing with each other and the second nucleic acid region forms a loop moiety.
(13) The production method according to (12), wherein the nucleic acid fragment is the double-stranded nucleic acid fragment (i) of (12), wherein the ligation step involves ligating the hairpin-shaped DNA to at least one modified nucleic acid strand of the nucleic acid fragment.
(14) The production method according to (12) or (13), wherein the first nucleic acid region consists of "g" or "c" base.
(15) The production method according to any of (12) to (14), wherein the modified nucleotide in the nucleic acid fragment is composed of RNA.
(16) The production method according to (15), wherein the modification is substitution of a 2'-hydroxy group in ribose.
(17) The production method according to (16), wherein the hydroxy group is substituted by a methoxy group, an ethoxy group, a propoxy group, or a butoxy group.

The present specification encompasses the contents described in the specification and/or drawings of Japanese Patent Application No. 2011-230090, which serves as a basis for the priority of the present application.

### Advantageous Effects of Invention

Use of the nucleic acid molecule of the present invention can impart resistance to degradation by a nucleolytic enzyme and high *in vivo* stability to a double-stranded nucleic acid fragment or a single-stranded nucleic acid fragment forming a higher-order structure via intramolecular folding in a simple and cost-effective manner. Thus, *in vivo* stability of functional nucleic acids contained in the double-stranded nucleic acid fragment or single-stranded nucleic acid fragment can be enhanced and its biological activity can be enhanced, and pharmacological effects thereof can be continuously exerted for a long period of time.

### Brief Description of Drawings

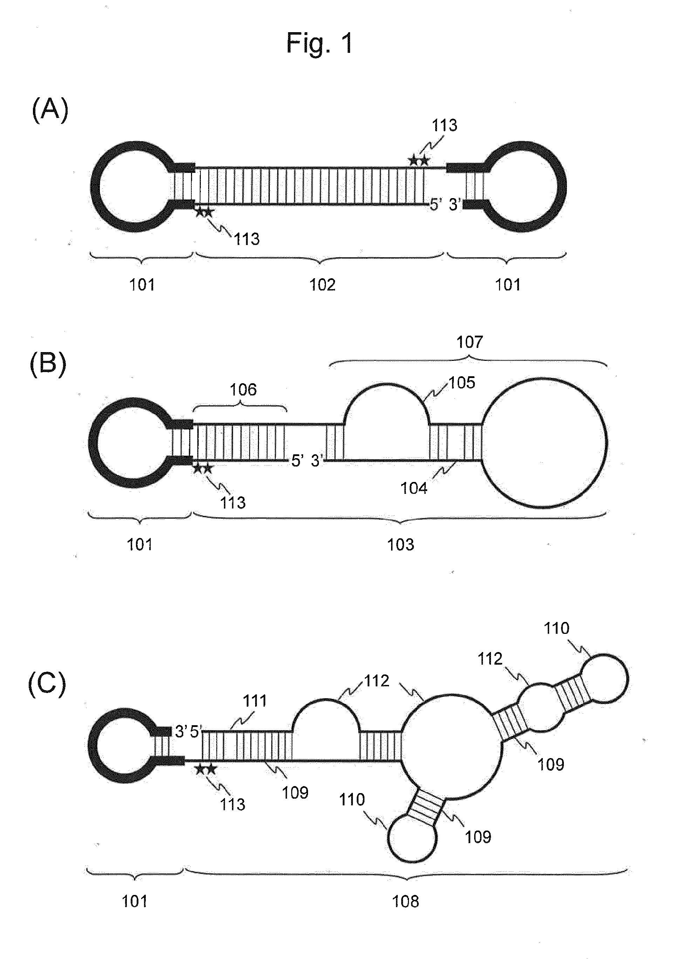

[Figure 1] Figure 1 is a conceptual diagram showing the nucleic acid molecule of the present invention; wherein (A) shows an example of a nucleic acid molecule consisting of two hairpin-shaped DNAs (101) and a double-stranded nucleic acid fragment (102) composed of strands of the same base length; (B) shows an example of a nucleic acid molecule consisting of one hairpin-shaped DNA (101) and a double-stranded nucleic acid fragment (103) composed of two strands of different base lengths; and (C) shows an example of a nucleic acid molecule consisting of one hairpin-shaped DNA (101) and a single-stranded nucleic acid fragment (108). In the figure, asterisks (113) indicate the position of a nucleotide that may be modified (the same holds true for the description below).

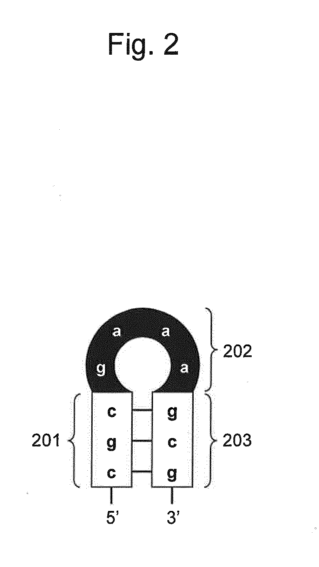

[Figure 2] Figure 2 is a conceptual diagram showing a hairpin-shaped DNA constituting the nucleic acid molecule of the present invention. The hairpin-shaped DNA comprises a first nucleic acid region (201), a second nucleic acid region (202), and a third nucleic acid region (203), and nucleotides constituting the first nucleic acid region (201) are base-paired with nucleotides constituting the third nucleic acid region (203) to form a stem structure.

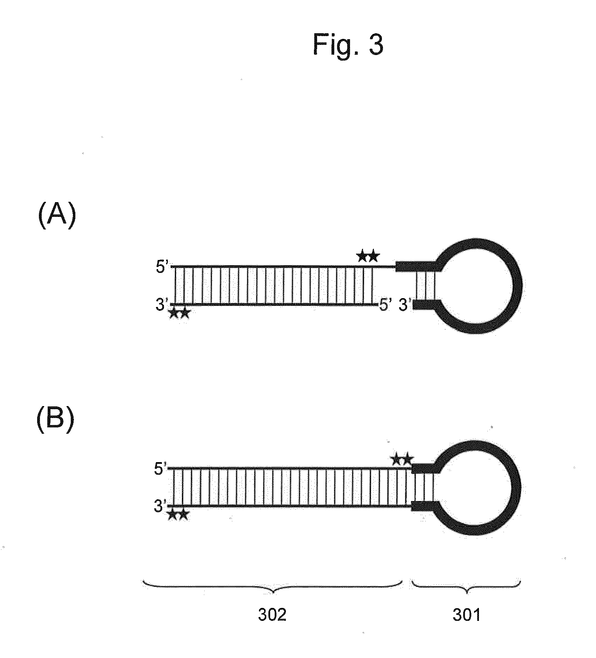

[Figure 3] Figure 3 is a conceptual diagram showing an embodiment of the nucleic acid molecule of the present invention consisting of a hairpin-shaped DNA (301) ligated to a double-stranded nucleic acid fragment (302). In the drawing, an asterisk indicates the position of a nucleotide which can be modified.

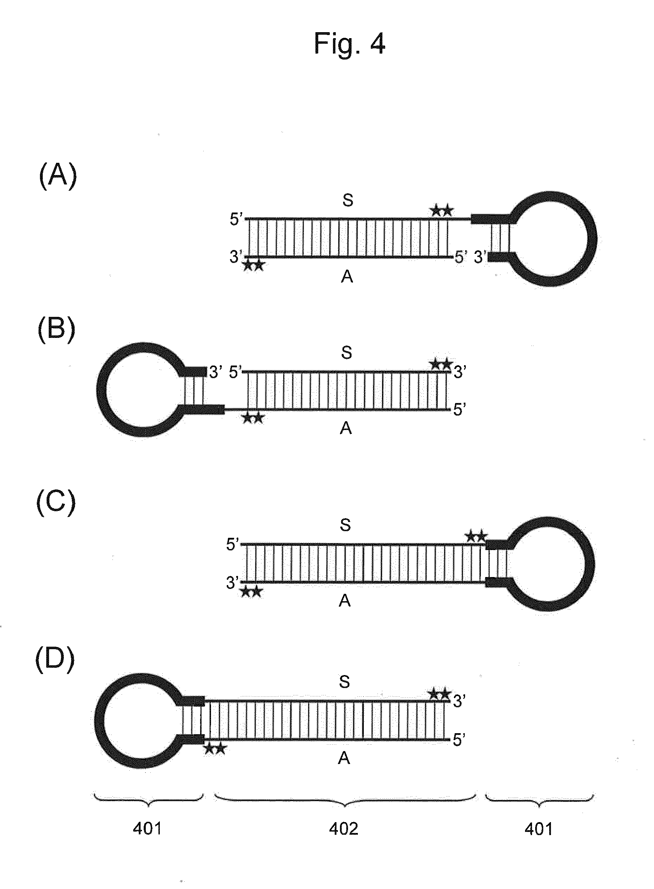

[Figure 4] Figure 4 is a conceptual diagram showing an embodiment of the nucleic acid molecule of the present invention consisting of a hairpin-shaped DNA (401) ligated to a double-stranded nucleic acid fragment (402), when the double-stranded nucleic acid fragment comprises an siRNA base sequence. In the double-stranded nucleic acid fragment (402), the S side is a nucleic acid fragment comprising an siRNA sense strand, and the A side is a nucleic acid fragment comprising an siRNA antisense strand.

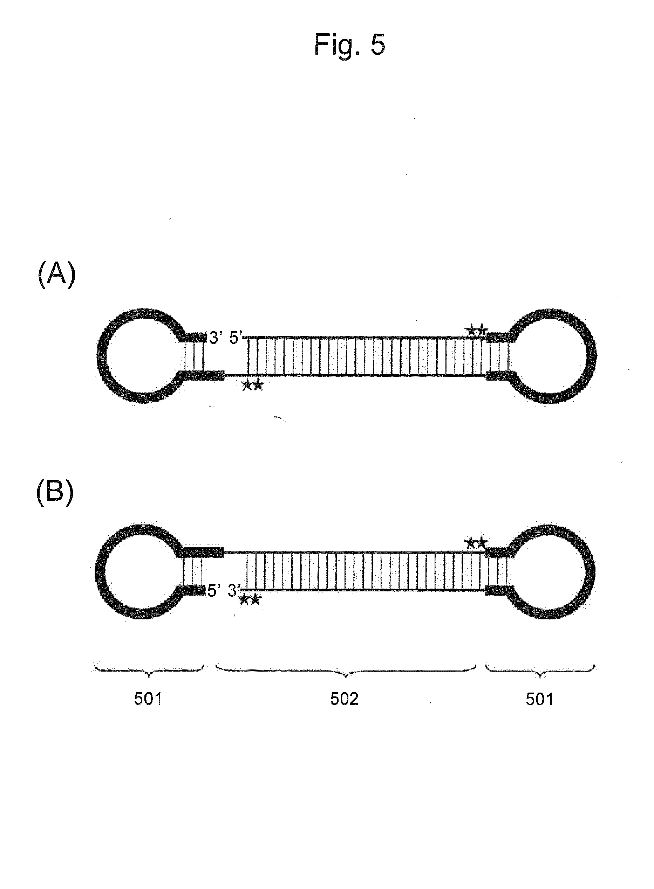

[Figure 5] Figure 5 is a conceptual diagram showing an embodiment of the nucleic acid molecule of the present invention consisting of two hairpin-shaped DNAs (501) ligated to a double-stranded nucleic acid fragment (502), when the nucleic acid comprises a non-ligated region.

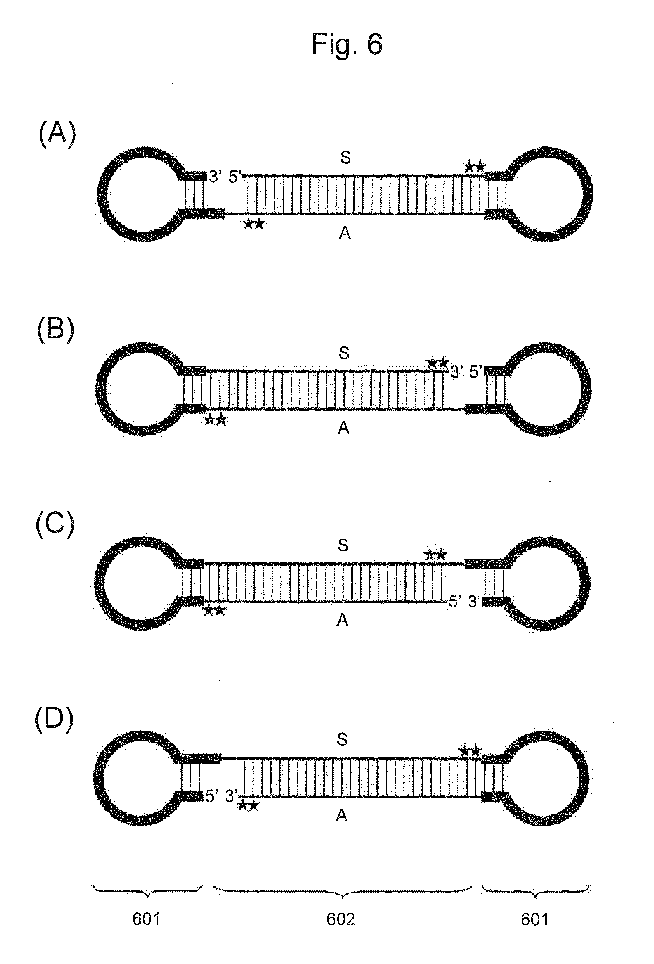

[Figure 6] Figure 6 is a conceptual diagram showing an embodiment of the nucleic acid molecule of the present invention consisting of two hairpin-shaped DNAs (601) ligated to a double-stranded nucleic acid fragment (602), when the nucleic acid comprises a non-ligated region and the double-stranded nucleic acid fragment comprises an siRNA base sequence. In the double-stranded nucleic acid fragment (602), the S side is a nucleic acid fragment comprising an siRNA sense strand, and the A side is a nucleic acid fragment comprising an siRNA antisense strand.

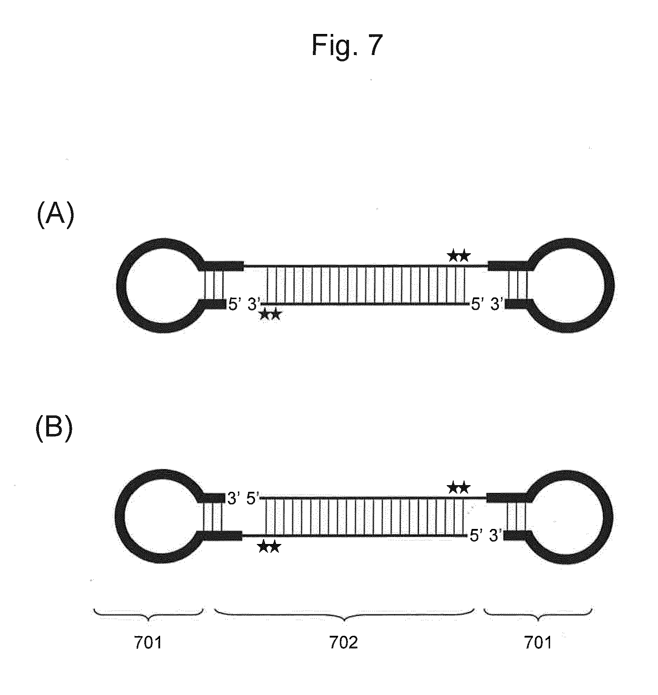

[Figure 7] Figure 7 is a conceptual diagram showing an embodiment of the nucleic acid molecule of the present invention consisting of two hairpin-shaped DNAs (701) ligated to a double-stranded nucleic acid fragment (702), when the nucleic acid comprises two non-ligated regions.

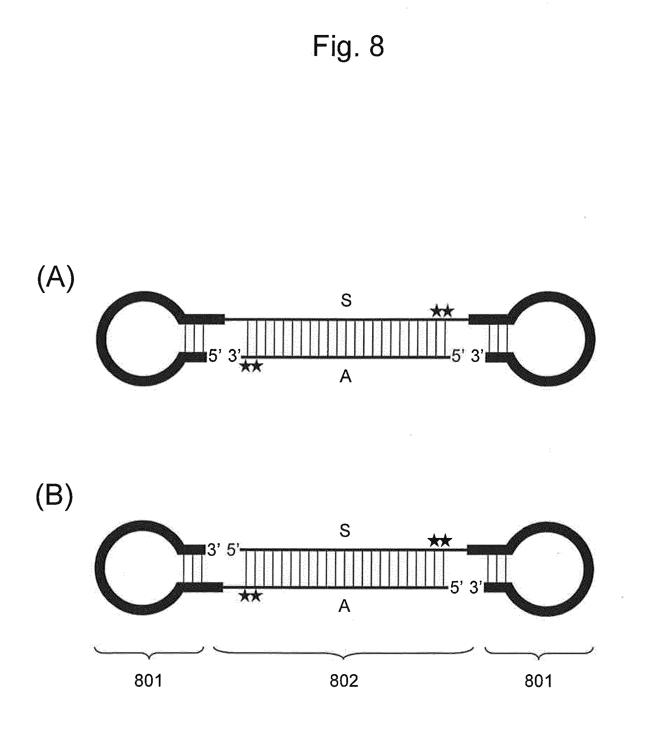

[Figure 8] Figure 8 is a conceptual diagram showing an embodiment of the nucleic acid molecule of the present invention consisting of two hairpin-shaped DNAs (801) ligated to a double-stranded nucleic acid fragment (802), when the nucleic acid comprises two non-ligated regions and the double-stranded nucleic acid fragment comprises an siRNA base sequence. In the double-stranded nucleic acid fragment (802), the S side is a nucleic acid fragment comprising an siRNA sense strand, and the A side is a nucleic acid fragment comprising an siRNA antisense strand.

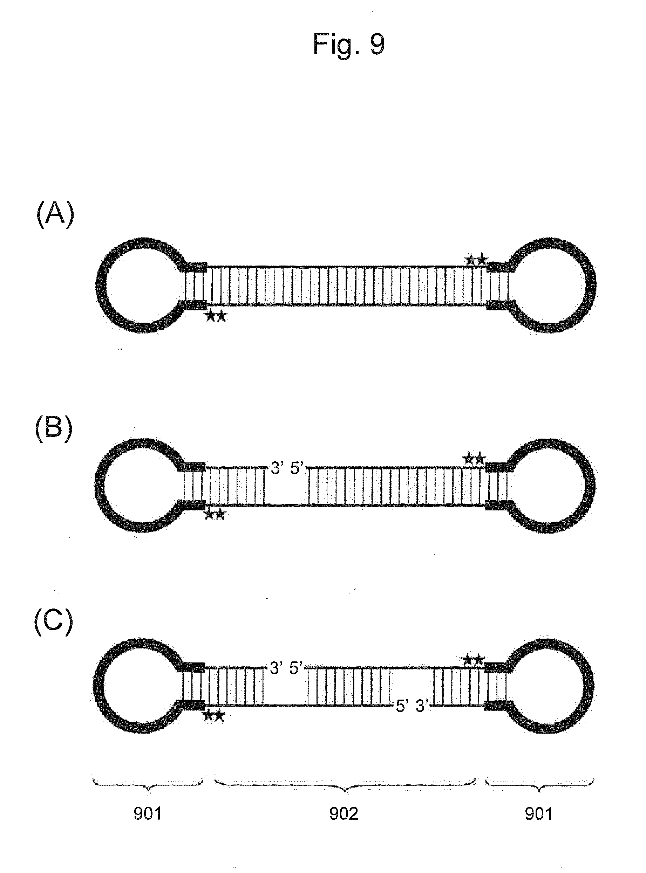

[Figure 9] Figure 9 is a conceptual diagram showing an embodiment of the nucleic acid molecule of the present invention, showing the number of nicks in a double-stranded nucleic acid fragment (902) and positions thereof, wherein all the ends of two hairpin-shaped DNAs (901) ligated to all the ends of the double-stranded nucleic acid fragment (902).

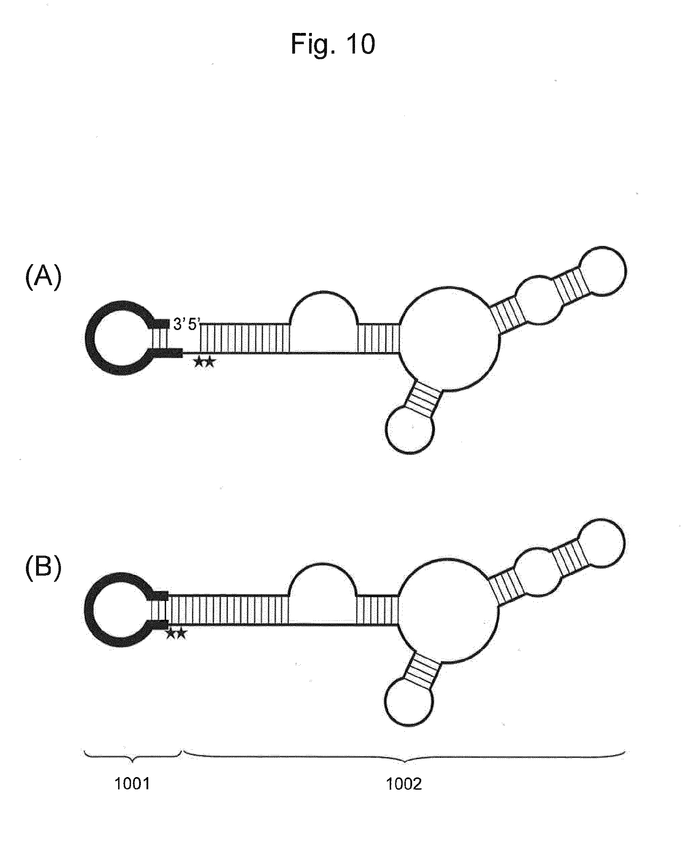

[Figure 10] Figure 10 is a conceptual diagram showing an embodiment of the nucleic acid molecule of the present invention consisting of a hairpin-shaped DNA (1001) ligated to a single-stranded nucleic acid fragment (1002).

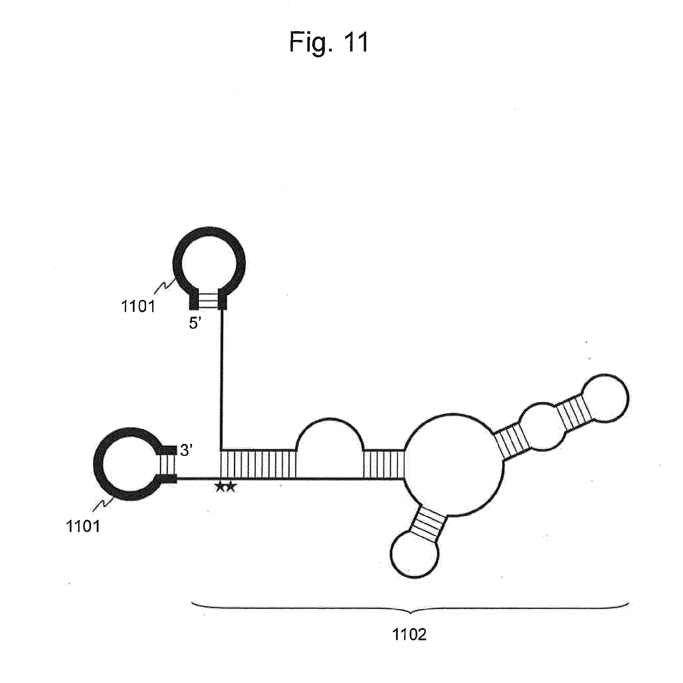

[Figure 11] Figure 11 is a conceptual diagram showing embodiments of the nucleic acid molecule of the present invention consisting of two hairpin-shaped DNAs (1101) ligated to a single-stranded nucleic acid fragment (1102).

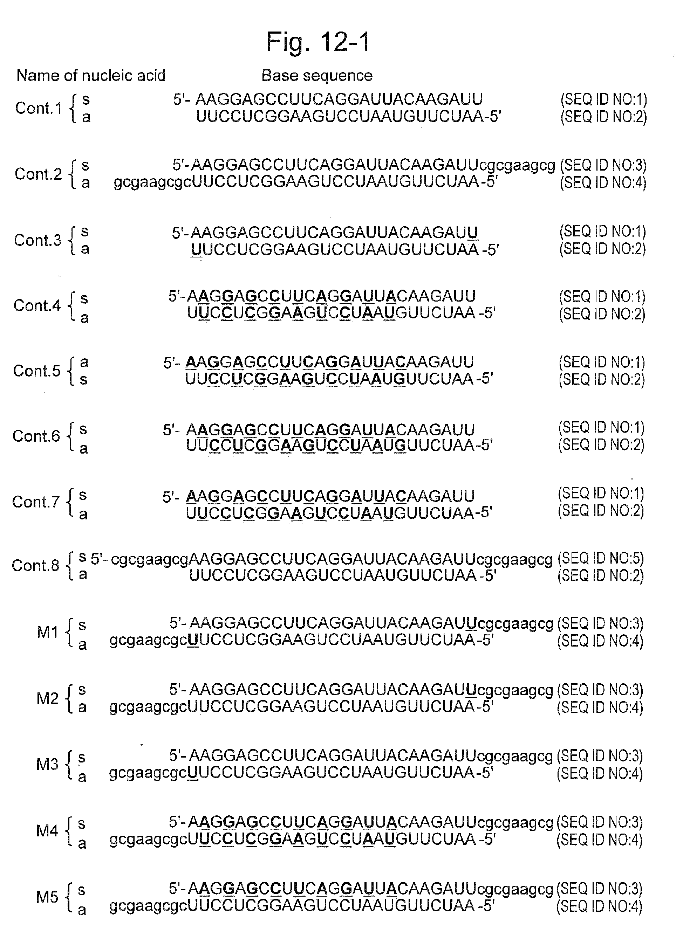

[Figure 12-1] Figure 12-1 shows molecular names of various nucleic acid sequence molecules, base sequences and SEQ ID NOs containing siRNA targeting firefly luciferase mRNA prepared in Example 1. DNA sequences are represented by lower-case letters and RNA sequences are represented by upper-case letters. An underlined boldface letter indicates a nucleotide comprising 2'-O-methylribose (2'OMe). An italicized boldface letter indicates a nucleotide comprising 2'-deoxy-2'-fluororibose (F). In each nucleic acid molecule, s indicates a nucleic acid fragment comprising an siRNA sense strand, and indicates a nucleic acid fragment comprising an siRNA antisense strand. Bases of the siRNA sense strand are base-paired with corresponding bases of the antisense strand. A DNA moiety corresponds to the hairpin-shaped DNA described herein and forms a hairpin structure shown in Figure 2.

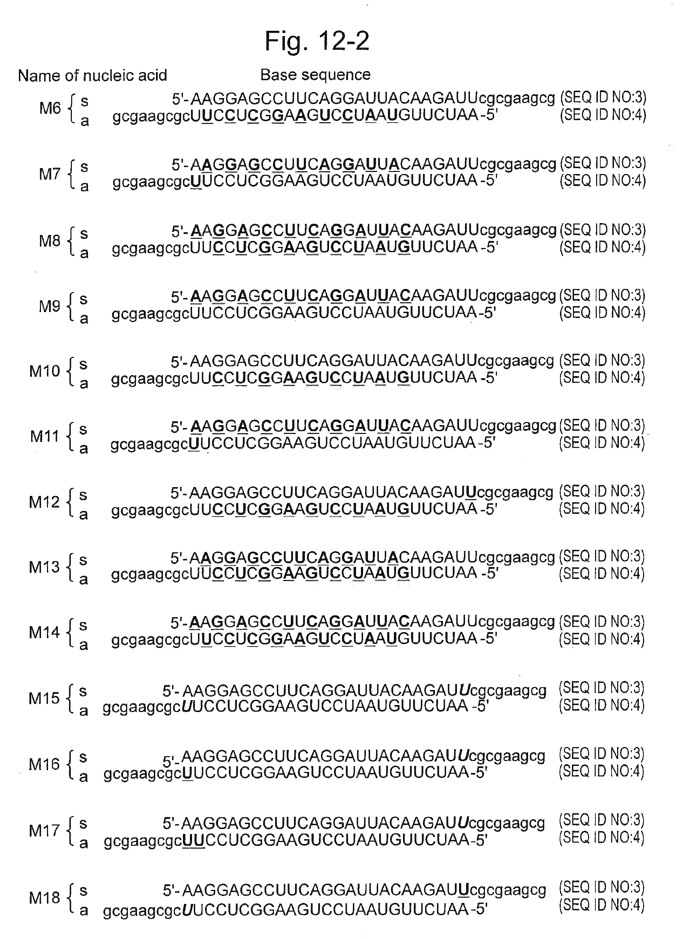

[Figure 12-2] Same as Figure 12-1.

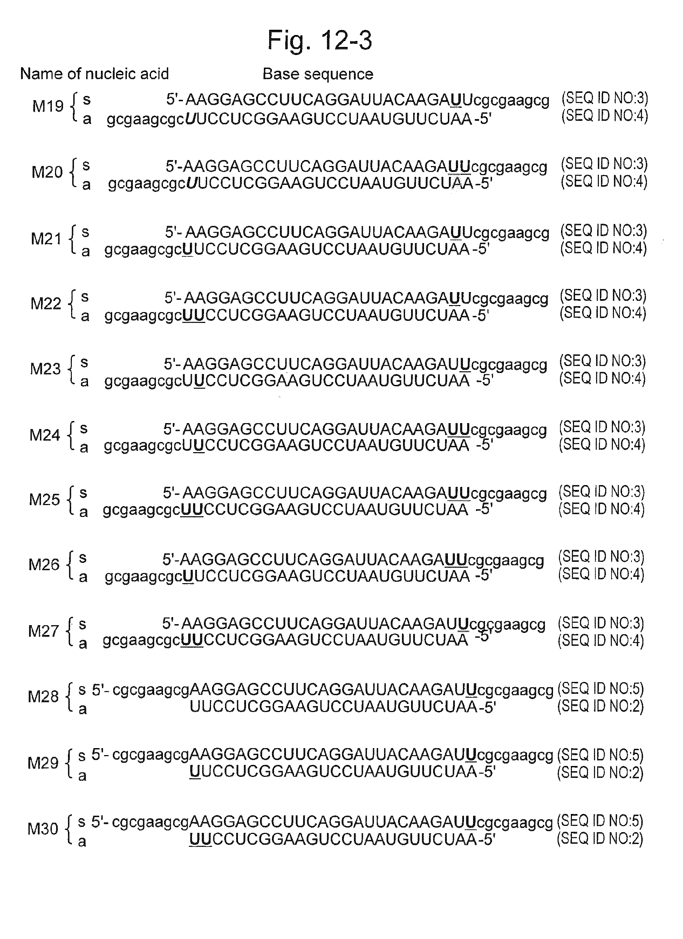

[Figure 12-3] Same as Figure 12-1.

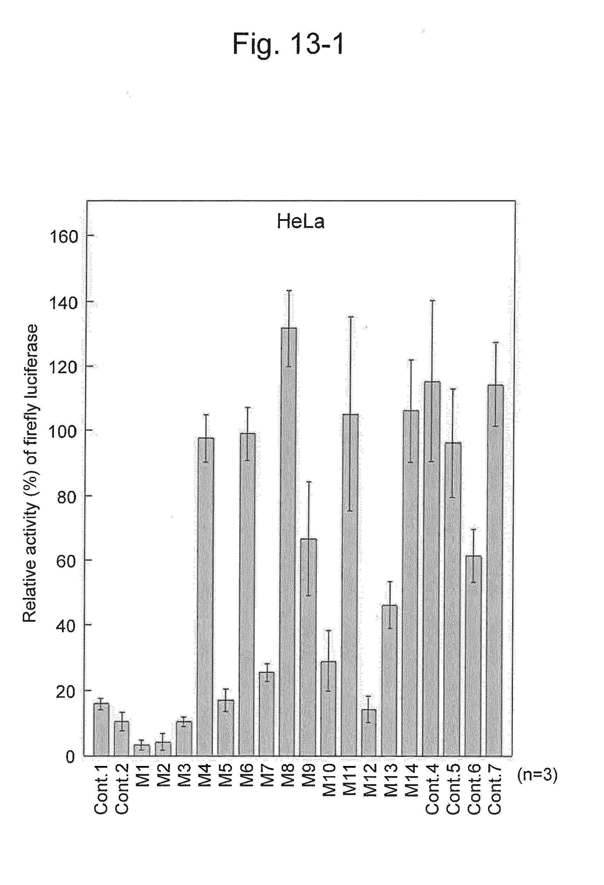

[Figure 13-1] Figure 13-1 shows the results of suppression of firefly luciferase expression by various nucleic acids containing firefly luciferase siRNAs in HeLa cells. The results show relative expression levels of firefly luciferase, corrected by the expression levels of *Renilla reniformis* luciferase, data not shown. The relative luciferase emission obtained when samples were not treated with siRNA-containing nucleic acids (without treatment) was designated as 100%. Here shown are the results about Cont. (control) 1, 2, and 4 to 7 as well as test nucleic acid molecules M1 to M14.

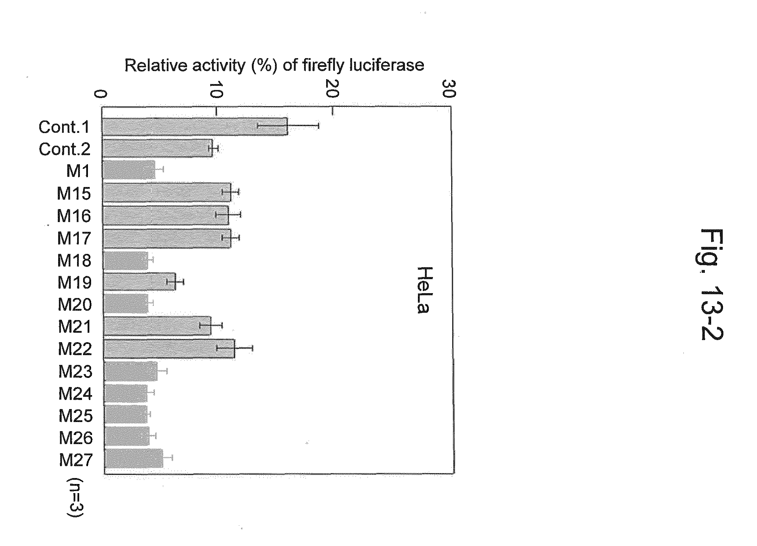

[Figure 13-2] Same as Figure 13-1. Here shown are the results about Cont. 1 and 2 as well as test nucleic acid molecules M1 and M15 to M27.

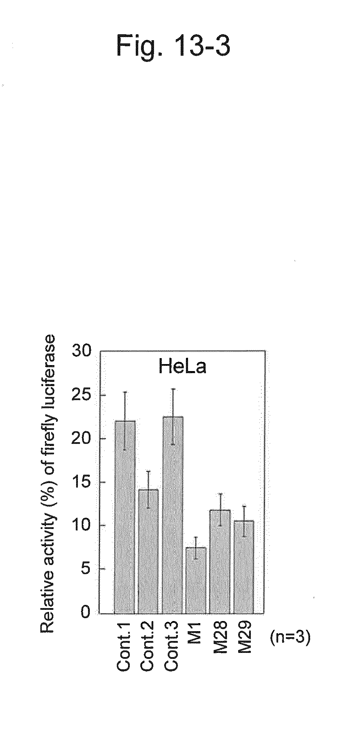

[Figure 13-3] Same as Figure 13-1. Here shown are the results about Cont. 1, 2, and 3 as well as test nucleic acid molecules M1, M28, and M29.

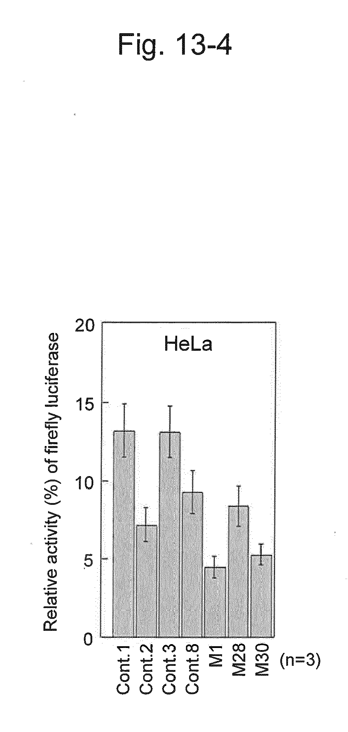

[Figure 13-4] Same as Figure 13-1. Here shown are the results about Cont. 1, 2, 3, and 8 as well as test nucleic acid molecules M1, M28, and M30.

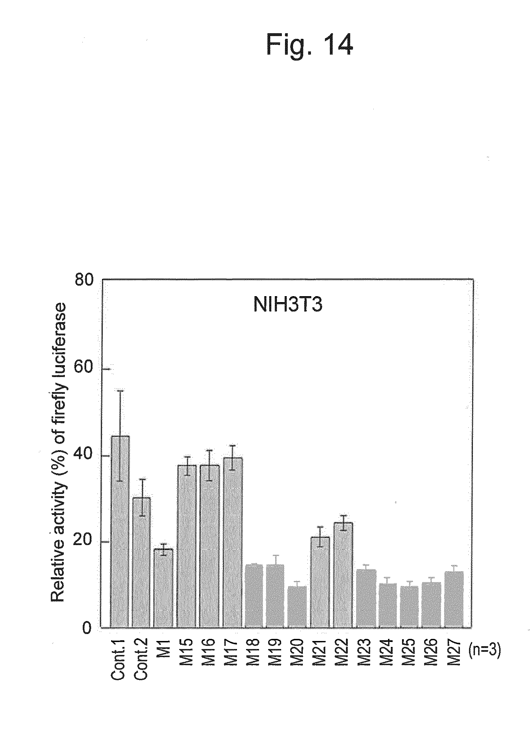

[Figure 14] Figure 14 shows the effects of suppression of firefly luciferase expression by various nucleic acids containing firefly luciferase siRNAs in NIH3T3 cells. The results show relative expression levels of firefly luciferase, corrected by the expression levels of *Renilla reniformis* luciferase (data not shown). The relative luciferase emission obtained when samples were not treated with siRNA-containing nucleic acids (without treatment) was designated as 100%. Here shown the results about Cont. 1 and 2, test nucleic acid molecule M1 as well as M15 to M27, which exhibited relatively high effects of suppression when HeLa cells were used.

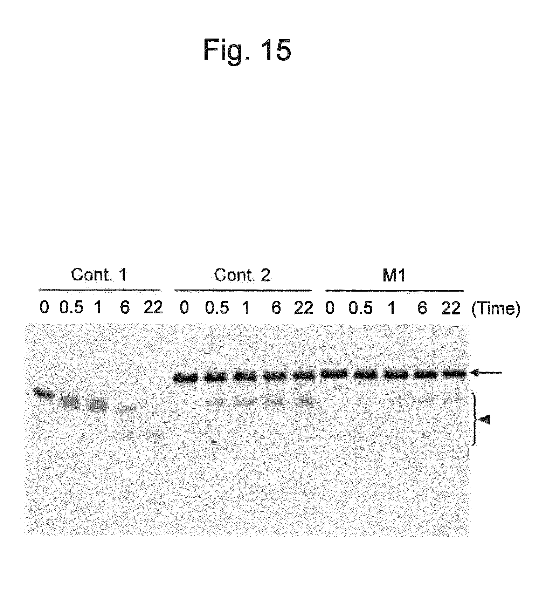

[Figure 15] Figure 15 is a denaturing polyacrylamide gel electrophoresis pattern showing the stability of each nucleic acid molecule of the present invention against a nucleolytic enzyme in mouse serum. In the figure, a band indicated by arrow indicates a position corresponding to a full-length nucleic acid fragment of the nucleic acid molecule of the present invention. A band group indicated by arrowhead indicates positions corresponding to nucleic acid fragments resulting from partial degradation of the nucleic acid molecule of the present invention.

### Description of Embodiments

### I. Nucleic acid molecule

The first aspect of the present invention relates to a nucleic acid molecule having resistance to degradation by a nucleolytic enzyme. Figure 1 is a diagram showing examples of structures of the nucleic acid molecule of the present invention. As shown in (A) to (C) of Figure 1, the nucleic acid molecule of the present invention comprises a hairpin-shaped DNA (101) and a nucleic acid fragment (102, 103, or 108), wherein the hairpin-shaped DNA is ligated to at least one 3' end of the nucleic acid fragment and wherein at least one of two 3' terminal nucleotides from at least one 3' end of the nucleic acid fragment is modified (113).

In principle, the term "nucleic acid" used herein refers to a biopolymer comprising, as constitutional units, nucleotides ligated to each other via a phosphodiester bond. In general, accordingly, the term refers to a naturally occurring nucleic acid to which a naturally occurring nucleotide existing in nature is ligated, such as DNA comprising deoxyribonucleotides having any of adenine, guanine, cytosine, and thymine ligated to each other and/or RNA comprising ribonucleotides having any of adenine, guanine, cytosine, and uracil ligated to each other. In addition, non-naturally occurring nucleotides and non-naturally occurring nucleic acids may be within the scope of the nucleic acid molecule of the present invention.

The term "non-naturally occurring nucleotide" used herein refers to an artificially constructed nucleotide, which does not exist in nature, having properties and/or structures similar to those of a naturally occurring nucleotide or comprising nucleosides or bases having properties and/or structures similar to those of nucleosides or bases constituting a naturally occurring nucleotide. Examples thereof include abasic nucleoside, arabinonucleoside, 2'-deoxyuridine, α-deoxyribonucleoside, and β-L-deoxyribonucleoside. The term "non-naturally occurring nucleic acid" used herein refers to an artificially constructed artificial nucleic acid having a structure and/or properties similar to those of a naturally occurring nucleic acid. Examples thereof include peptide nucleic acids (PNA), peptide nucleic acids with phosphate groups (PHONA), bridged nucleic acids/locked nucleic acids (BNA/LNA), and morpholino nucleic acids. Further examples include chemically-modified nucleic acids and artificial nucleic acids such as methylphosphonate DNA/RNA, phosphorothioate DNA/RNA, phosphoramidate DNA/RNA, and 2'-O-methyl DNA/RNA. Hereinafter, non-naturally occurring nucleotides and non-naturally occurring nucleic acids are collectively referred to as "artificial nucleic acids" for convenience.

A phosphoric acid group, a sugar, and/or a base in the nucleic acid molecule of the present invention may be labeled as necessary. Any substances for nucleic acid labeling known in the art can be used for labeling. Examples thereof include radioactive isotopes (e.g., ³²P, ³H, and ¹⁴C), DIG, biotin, fluorescent dyes (e.g., FITC, Texas, cy3, cy5, cy7, FAM, HEX, VIC, JOE, Rox, TET, Bodily493, NBD, and TAMRA), and luminescent substances (e.g., acridinium ester).

### 1. Constitutional element of the nucleic acid molecule

As described above, the nucleic acid molecule of the present invention is composed of a hairpin-shaped DNA (101) and nucleic acid fragments (i.e., a double-stranded nucleic acid fragment 102 or 103, or a single-stranded nucleic acid fragment 108). Hereafter, these constituents are described in detail.

### 1-1. Hairpin-shaped DNA

Figure 2 shows an example of hairpin-shaped DNA constituting the nucleic acid molecule of the present invention. As shown in Figure 2, a hairpin-shaped DNA comprises three DNA nucleic acid regions: the first nucleic acid region (201); the second nucleic acid region (202); and the third nucleic acid region (203), sequentially ligated from the 5' end toward the 3' end.

The "first nucleic acid region" comprises 2 to 5 arbitrary nucleotides. The term "nucleotide" refers to a "deoxyribonucleotide" having guanine (g), adenine (a), cytosine (c), or thymine (t). A nucleotide in such nucleic acid region is preferably guanine or cytosine for the following reason: when the first nucleic acid region forms a stem structure with the third nucleic acid region, a larger "gc" content results in the increased Tm value, and the stem structure can then be stably maintained. Accordingly, it is most preferable that the entire base sequence of the first nucleic acid region be composed of "g" and/or "c". In an example of Figure 2, the first nucleic acid region is shown as 5'-cgc-3'.

The "second nucleic acid region" comprises the 5'-gna-3' or 5'-gnna-3' base sequence. Each "n" in the sequence is independently composed of any naturally occurring base (g, a, t, or c), the base analogue, or the modified base. In an example of Figure 2, the second nucleic acid region is shown as 5'-gaaa-3'.

The "third nucleic acid region" has a base sequence complementary to the first nucleic acid region. Accordingly, the base sequence of the third nucleic acid region is determined based on the base sequence of the first nucleic acid region, and the first nucleic acid region and the third nucleic acid region form base-pairing with each other in the molecule. As a result, the first nucleic acid region and the third nucleic acid region constitute a stem moiety in which all bases are paired with each other. The second nucleic acid region located between the first nucleic acid region and the third nucleic acid region forms loop moiety therewith. For example, the hairpin-shaped DNA consisting of 7 to 14 nucleotides having the base sequence as shown in SEQ ID NO: 6 or 7 is formed as a whole.

Such hairpin-shaped DNA may be ligated to at least one end of the nucleic acid fragment described below via phosphodiester linkage. Thus, resistance of the nucleic acid fragment to degradation by the nucleolytic enzyme can be enhanced, and stability thereof can be enhanced *in vivo.*

### 1-2. Nucleic acid fragment

A nucleic acid fragment constituting the nucleic acid molecule of the present invention is a double-stranded nucleic acid fragment or a single-stranded nucleic acid fragment forming a higher-order structure via intramolecular folding. In either nucleic acid fragment, the hairpin-shaped DNA is ligated to at least one 3' end, while at least one of two 3' terminal nucleotides from at least one 3' end is modified.

Such nucleic acid fragment can include a base sequence of a functional nucleic acid. The term "functional nucleic acid" used herein refers to a nucleic acid having specific biological functions *in vivo* or in cells, such as enzymatic functions, catalytic functions, or biologically inhibiting or enhancing functions (e.g., inhibition or enhancement of transcription or translation). Specific examples include siRNA, shRNA, miRNA (including pri-miRNA and pre-miRNA), nucleic acid aptamers (including RNA aptamers and DNA aptamers), ribozymes (including deoxyribozymes), riboswitches, U1 adaptors, molecular beacons, and transcriptional factor-binding regions. In the present invention, the functional nucleic acid is preferably an RNA-derived functional nucleic acid, for example, siRNA, shRNA, or miRNA.

Hereafter, the double-stranded nucleic acid fragment and the single-stranded nucleic acid fragment are described in detail.

### 1-2-1. Double-stranded nucleic acid fragment

A double-stranded nucleic acid fragment is composed of DNA, RNA, or an artificial nucleic acid, or a combination thereof. Bases in nucleic acid fragments constituting a double-stranded nucleic acid fragment are completely or partially base-paired in the nucleic acid molecule of the present invention. The term "completely" used herein refers to a condition in which all bases of at least one nucleic acid fragment are base-paired with corresponding bases of another nucleic acid fragment. When the base lengths of the nucleic acid fragments are equivalent, accordingly, all the bases of the two nucleic acid fragments are base-paired. The term "partially" used herein refers to a condition in which some, and preferable at least two continuous bases in the relevant base sequences of nucleic acid fragments are base-paired. In such a case, accordingly, the double-stranded nucleic acid fragment may comprise a mismatched region (104) of one or more bases or one or more bulge structures (105), as shown in Figure 1 (B).

The double-stranded nucleic acid fragment of the present invention comprises 3 or more, preferably 5 or more, more preferably 7 or more, and further preferably 10 or more base pairs as a whole. The base length of the double-stranded nucleic acid fragment of the present invention is not particularly limited. Such length may be adequately determined, so that each functional nucleic acid region can exert its functions. Base lengths of the nucleic acid fragments constituting a double-stranded nucleic acid fragment may be the same or different. The same base length is preferable. When base lengths of the nucleic acid fragments are different from each other, a longer strand may form one or more loop structures and one or more stem structures (107) via intramolecular folding, as shown in Figure 1 (B). In such a case, a stem structure can contain one or more mismatched regions (104) or one or more bulge structures (105).

The double-stranded nucleic acid fragment has two 3' ends, at least one of which is constituted such that the hairpin-shaped DNA is ligated thereto. In addition, at least one of these two 3' ends of the nucleic acid fragment is modified. The hairpin-shaped DNA may or may not be ligated to this modified nucleic acid strand of the nucleic acid fragment. This means that when one of two nucleic acid fragments constituting the double-stranded nucleic acid fragment is modified, the hairpin-shaped DNA may be ligated to the 3' end of only this modified nucleic acid fragment, may be ligated to the 3' end of only the unmodified nucleic acid fragment, or may be ligated to each of the 3' ends of the modified nucleic acid fragment and the unmodified nucleic acid fragment. Alternatively, when two nucleic acid fragments constituting the double-stranded nucleic acid fragment are both modified, the hairpin-shaped DNA may be ligated to the 3' end of only one of the nucleic acid fragments or may be ligated to the 3' ends of both the nucleic acid fragments. According to a preferred embodiment, at least one hairpin-shaped DNA-ligated nucleic acid strand of the nucleic acid fragment is modified. This means that when only one of two nucleic acid fragments constituting the double-stranded nucleic acid fragment is modified, the hairpin-shaped DNA is preferably ligated to the 3' end of at least the modified nucleic acid fragment (i.e., the hairpin-shaped DNA may or may not be ligated to the 3' end of the other nucleic acid fragment (unmodified nucleic acid fragment)). Alternatively, when two nucleic acid fragments constituting the double-stranded nucleic acid fragment are both modified, the hairpin-shaped DNA is preferably ligated to the 3' end(s) of one or both of the nucleic acid fragments. Examples of the modification for the nucleic acid fragment include 3 types of modifications: modification of only the second nucleotide counted from the 3' end, modification of only the nucleotide at the 3' end, and modification of both two 3'-terminal nucleotides. When one nucleotide is modified, the nucleotide to be modified may be either the second nucleotide counted from the 3' end or the nucleotide at the 3' end. Preferably, the nucleotide at the 3' end is modified. When both the nucleotides are modified, each of the nucleotides may undergo the same modification or may under different modifications. The 5' end(s) may be constituted such that the hairpin-shaped DNA is ligated thereto or may be constituted such that the hairpin-shaped DNA is not ligated thereto.

The "modification" used herein refers to modification of a nucleic acid, wherein some or all of nucleotides as constitutional units of the nucleic acid are substituted by other atomic groups, or functional groups or the like are added to some or all of the nucleotides. Specific examples include sugar modification, base modification, and phosphate modification.

The "sugar modification" refers to modification of a ribose moiety constituting nucleoside. Examples thereof include modification of a ribose moiety constituting ribonucleoside, which is substitution of or addition to a 2'-hydroxy group. Specifically, such modification corresponds to, for example, substitution of the hydroxy group by a methoxy group that results in 2'-O-methylribose (hereinafter, 2'-O-methylribose is also abbreviated to "2'OMe"), substitution of the hydroxy group by an ethoxy group that results in 2'-O-ethylribose, substitution of the hydroxy group by a propoxy group that results in 2'-O-propylribose, substitution of the hydroxy group by a butoxy group that results in 2'-O-butylribose, substitution of the hydroxy group by a fluoro group that results in 2'-deoxy-2'-fluororibose (hereinafter, 2'-deoxy-2'-fluororibose is also abbreviated to "F"), or substitution of the hydroxy group by a 2'-O-methoxy-ethyl group that results in 2'-O-methoxyethylribose. Alternative examples include substitution of a (deoxy)ribose moiety of nucleoside by a different sugar. Specifically, such substitution corresponds to, for example, substitution of the ribose moiety by arabinose, 2'-fluoro-β-D-arabinose, a ribose derivative in which a 2'-hydroxy group and a 4'-carbon atom of ribose are cross-linked by methylene, or a ribose derivative in which 4'-oxygen in the ribose ring is substituted by sulfur. Also, substitution of an oxygen atom on a ribofuranose ring (4'-oxygen atom of ribose) by sulfur is included therein. In the case of the sugar modification, the nucleotide to be modified is not limited by its base. For example, DNA may have any base selected from guanine, adenine, cytosine, and thymine. Alternatively, RNA may have any base selected from guanine, adenine, cytosine, and uracil.

The "base modification" refers to modification of a base moiety constituting nucleoside. Examples thereof include substitution of the base moiety by a functional group, addition of a functional group to the base moiety, and substitution of the base moiety by a base analogue. Specifically, such a modified base corresponds to, for example, modified pyrimidines such as 5-methylcytosine resulting from substitution at position 5 of cytosine by a methyl group, 5-hydroxycytosine resulting from substitution at position 5 of cytosine by a hydroxy group, 5-fluorouracil resulting from substitution at position 5 of uracil by a fluoro group, 4-thiouracil resulting from substitution of a 4-oxygen atom of uracil by a thio group, 5-methyluracil resulting from substitution at position 5 of uracil by a methyl group, and 2-thiouracil resulting from substitution of a 2-oxygen atom of uracil by a thio group, modified purines such as 6-methyladenine resulting from substitution at position 6 of adenine by a methyl group, and 6-thioguanine resulting from substitution at position 6 of guanine by a thio group, or other heterocyclic bases. The base analogue corresponds to, for example, a 2-oxo(1H)-pyridin-3-yl group, a 5-substituted 2-oxo(1H)-pyridin-3-yl group, a 2-amino-6-(2-thiazolyl)purin-9-yl group, a 2-amino-6-(2-thiazolyl)purin-9-yl group, a 2-amino-6-(2-oxazolyl)purin-9-yl group, a 2,4-difluoro-5-methylbenzyl group, or a 7-(2,2'-bithien-5-yl)imidazo[4,5-b]pyridin-3-yl group.

As described above, the double-stranded nucleic acid fragment can contain a base sequence of a functional nucleic acid. Examples of functional nucleic acids that can be contained in the double-stranded nucleic acid fragment include siRNA, mature double-stranded miRNA, and target molecule-binding nucleic acid fragment. These members are described below.

### <siRNA>

Small interference RNA (siRNA) is small-molecular double-stranded RNA consisting of a sense strand having a base sequence corresponding to a part of a target gene (passenger strand) and an antisense strand thereof (guide strand). siRNA can induce sequence-specific post-transcriptional gene silencing (i.e., RNA interference) by introducing it into cells (eukaryotic cells) (Fire A. et al., 1998, Nature, 391, 806-811). Hereafter, a case in which a siRNA base sequence is contained in a double-stranded nucleic acid fragment constituting the nucleic acid molecule of the present invention is described.

siRNA contained in the double-stranded nucleic acid fragment includes a base sequence that completely matches with a continuous partial region of the base sequence of a sense strand of a target gene in either nucleic acid fragment. The length of the completely matched base sequence is 17 to 32 bases, preferably 18 to 30 bases, and more preferably 19 to 25 bases.

A nucleic acid region corresponding to siRNA in the double-stranded nucleic acid fragment is composed of RNA in principle. Such region can contain one or several artificial nucleic acids. The term "several" used herein refers to an integer between 2 and 30, such as integers between 2 and 30, 2 and 29, 2 and 28, 2 and 27, 2 and 26, 2 and 25, 2 and 24, 2 and 23, 2 and 22, 2 and 21, 2 and 20, 2 and 19, 2 and 18, 2 and 17, 2 and 16, 2 and 15, 2 and 14, 2 and 13, 2 and 12, 2 and 11, 2 and 10, 2 and 9, 2 and 8, 2 and 7, 2 and 6, 2 and 5, 2 and 4, and 2 and 3.

In the base sequence of the double-stranded nucleic acid fragment, at least one nucleotide (DNA and/or RNA) or artificial nucleic acid may be present at one or both ends of siRNA. The number of nucleotides or the like at one or both ends of siRNA is not particularly limited. If such end is ligated to the hairpin-shaped DNA, the number is preferably between 1 and 20. Specifically, thymine-thymine (TT) or uracil-uracil (UU) can be added to the 3' end of the siRNA sense strand and that of the RNA antisense strand, for example.

The target gene of siRNA is not particularly limited. Accordingly, the nucleic acid molecule of the present invention can include an siRNA base sequence corresponding to any target gene, in principle.

siRNA may be designed in accordance with a conventional technique based on a target gene base sequence. For example, the sequence can be designed based on the method of Ui-Tei et al. (Nucleic Acids Res., 32: 936-948, 2004), the method of Reynolds et al. (Nat. Biotechnol., 22: 326-330, 2004), or the method of Amarzguioui et al. (Biochem. Biophys. Res. Commun., 316: 1050-1058, 2004). In addition, web sites on which siRNA can be designed have been made available to public by a variety of research institutes or companies, and effective siRNA can be designed on the web. Representative examples of siRNA designing web sites include siDirect (http://design.RNAi.jp/), siSearch (http://www.epigeneticstation.com/epigenetic-links/detail/link-203.html), the siDESIGN Center (http://www.dharmacon.com/designcenter/designcenterpage.aspx), the siRNA Selection Server (http://jura.wi.mit.edu/bioc/siRNAext/), and the Gene Specific siRNA Selector (http://bioinfo.wistar.upenn.edu/siRNA/siRNA.htm).

In the double-stranded nucleic acid fragment containing the siRNA used herein, the hairpin-shaped DNA is ligated to the 3' end of at least one of a nucleic acid fragment comprising a sense strand and a nucleic acid fragment comprising an antisense strand. In addition, one or both of two 3' terminal nucleotides from the 3' end of at least one of the nucleic acid fragment comprising a sense strand and the nucleic acid fragment comprising an antisense strand are modified as mentioned above. When the nucleotide(s) of only one nucleic acid fragment is modified, the nucleic acid fragment to be modified may be either of the nucleic acid fragment comprising a sense strand or the nucleic acid fragment comprising an antisense strand. Preferably, the nucleic acid fragment comprising a sense strand is modified. This is because the results of research by the present inventors showed that the modification of the 3' end of the nucleic acid fragment comprising a sense strand offered stronger stability and resistance to degradation by a nucleolytic enzyme as well as high inhibitory effects on gene expression. Any types of modification may be made on the double-stranded nucleic acid fragment containing siRNA. For example, any of sugar modification and base modification can be performed. Sugar modification is preferred. Specific examples include modification with 2'OMe or F.

The nucleic acid molecule of the present invention comprising, as a constitutive element, a double-stranded nucleic acid fragment including such siRNA base sequence is capable of silencing the target gene expression *in vivo* or in cells with greater stability than conventional siRNA.

Also, the double-stranded nucleic acid fragment can include other functional nucleic acids, in addition to siRNA. When a nucleic acid fragment of the double-stranded nucleic acid fragment contains a long region that is not base-paired with the other nucleic acid fragment and one or more loop structures and one or more stem structures are consequently formed therein via intramolecular folding (i.e., the case as shown in Figure 1 (B) 107), for example, the double-stranded nucleic acid fragment may contain siRNA in a region in which a nucleic acid fragment is base-paired (106), and the double-stranded nucleic acid fragment may further contain other functional nucleic acids, such as RNA aptamers or single-stranded miRNA precursors, in a region in which a secondary structure is formed via intramolecular folding (107).

### <Mature double-stranded miRNA>

miRNA (micro RNA) is single-stranded non-coding RNA that is 21 to 23 bases in length, is present *in vivo,* and regulates the expression of a given gene. Such RNA is known to form a complex by binding to mRNA of a target gene and a protein factor and to inhibit the translation of the target gene. miRNA is transcribed from the genome as a single-stranded precursor referred to as pri-miRNA, further processed into a single-stranded precursor referred to as pre-miRNA with the use of an endonuclease referred to as Drosha in the nucleus, and converted into mature double-stranded miRNA consisting of miRNA strand and miRNA star strand by the action of an endonuclease referred to as Dicer outside the nucleus. miRNA strand is incorporated into an RISC (RNA-induced silencing complex) and it regulates the expression of the target gene as a mature single-stranded miRNA. A double-stranded nucleic acid fragment of the nucleic acid molecule of the present invention can include a base sequence of such mature double-stranded miRNA.

Mature double-stranded miRNA of a double-stranded nucleic acid fragment preferably has a base sequence identical to that of wild-type mature double-stranded miRNA. In such a case, such sequence may be designed based on the base sequence of miRNA encoded in the genome.

At least one nucleotide (DNA and/or RNA) or an artificial nucleic acid may be present at one or both ends of mature double-stranded miRNA in a double-stranded nucleic acid fragment. The number of nucleotides or the like at one or both ends of mature double-stranded miRNA is not particularly limited. If such end is to be ligated to the aforementioned hairpin-shaped DNA, such number is preferably between 1 and 20.

The target gene of mature double-stranded miRNA is not particularly limited. With the use of the nucleic acid molecule of the present invention, accordingly, miRNA of a gene of interest can be used when it is present in the genome.

Also in the double-stranded nucleic acid fragment containing mature double-stranded miRNA, as in the nucleic acid fragment containing siRNA, the hairpin-shaped DNA is ligated to the 3' end of at least one of a nucleic acid fragment comprising an miRNA strand and a nucleic acid fragment comprising an miRNA star strand. In addition, one or both of two 3' terminal nucleotides from the 3' end of at least one of the nucleic acid fragment comprising an miRNA strand and the nucleic acid fragment comprising an miRNA star strand are modified. When the nucleotide(s) of only one nucleic acid fragment is modified, either of the nucleic acid fragment comprising an miRNA strand or the nucleic acid fragment comprising an miRNA star strand may be modified. Preferably, the nucleic acid fragment comprising an miRNA strand is modified.

### <Target molecule-binding nucleic acid fragment>

The term "target molecule-binding nucleic acid fragment" refers to a nucleic acid fragment comprising binding region of a target molecule. The term "target molecule-binding region" refers to a region in a nucleic acid that can bind to a given target molecule with specificity and/or high affinity. A target molecule-binding nucleic acid fragment having a target molecule-binding region binds to a target molecule *in vivo* or in a cell to inhibit or suppress biological functions of the target molecule. Examples of target molecules include protein factors specifically binding to DNA or RNA, nucleic acids, and low-molecular-weight compounds. An example of a target molecule-binding nucleic acid fragment is a decoy DNA that has a DNA-binding region of a given transcription regulator and inhibits or suppresses functions of a transcription factor.

At least one nucleotide (DNA and/or RNA) or an artificial nucleic acid may be present at one or both ends of a target molecule-binding nucleic acid fragment in a base sequence of a double-stranded nucleic acid fragment. The number of nucleotides or the like at one or both ends is not particularly limited. If such end is to be ligated to the aforementioned hairpin-shaped DNA, such number is preferably between 1 and 20.

The target molecule of a target molecule-binding nucleic acid fragment is not particularly limited. If a binding region of the target molecule of interest is known, the target molecule may be designed and constructed based thereon.

Also in the double-stranded nucleic acid fragment containing a target molecule-binding nucleic acid fragment, as in the nucleic acid fragment containing siRNA, the hairpin-shaped DNA is ligated to the 3' end of at least one of two nucleic acid fragments constituting the nucleic acid fragment. In addition, one or both of two 3' terminal nucleotides from the 3' end of at least one of two nucleic acid fragments constituting the nucleic acid fragment are modified.

The nucleic acid molecule of the present invention comprising, as a constitutional element, a double-stranded nucleic acid fragment including a base sequence of such target molecule-binding nucleic acid fragment is capable of inhibiting or suppressing biological functions of a target molecule *in vivo* or in a cell with greater activity and stability than existing target molecule-binding nucleic acid fragments.

### 1-2-2. Single-stranded nucleic acid fragment,

A single-stranded nucleic acid fragment constituting the nucleic acid molecule of the present invention has at least one stem structure (109) and at least one loop structure (110) resulting from intramolecular folding, as shown in Figure 1 (C). Further, a stem structure may contain at least one mismatched region (111) and/or at least one bulge structure (112).

The single-stranded nucleic acid fragment has only one 3' end. This 3' end is therefore constituted such that the hairpin-shaped DNA is ligated thereto without exception. Hence, at least one of two 3' terminal nucleotides is modified as mentioned above. When one nucleotide is modified, the nucleotide to be modified is either of the second nucleotide counted from the 3' end or the nucleotide at the 3' end. Preferably, the nucleotide at the 3' end is modified. Alternatively, when two nucleotides are modified, these nucleotides may undergo the same modification or may undergo different modifications. The 5' end may be constituted such that the hairpin-shaped DNA is ligated thereto or may be constituted such that the hairpin-shaped DNA is not ligated thereto.

A functional nucleic acid that can be contained in the single-stranded nucleic acid fragment forms a higher-order structure via intramolecular folding and exerts its functions. Examples include shRNA, a single-stranded miRNA precursor, a nucleic acid aptamer, a ribozyme (including deoxyribozyme), a molecular beacon, a riboswitch, a U1 adaptor, and a target molecule-binding nucleic acid fragment having a target molecule-binding region. A functional nucleic acid that does not generally form a higher-order structure via intramolecular folding, such as a primer, probe, mature single-stranded miRNA, or antisense DNA, is not contained in the single-stranded nucleic acid fragment. Hereafter, a functional nucleic acid that can be contained in a single-stranded nucleic acid fragment is described.

### <shRNA>

shRNA (short hairpin RNA) is single-stranded RNA comprising siRNA or mature double-stranded miRNA ligated by an adequate short spacer sequence having a sequence. Accordingly, a sense region is base-paired with an antisense region to form a stem structure in a molecule, and the spacer sequence forms a loop structure therein. Thereby, an shRNA molecule forms a hairpin-shaped stem-loop structure as a whole. A spacer sequence generally comprises 3 to 24 bases and preferably 4 to 15 bases. A spacer sequence is not particularly limited, provided that siRNA or mature double-stranded miRNA is capable of base pairing.

At least one nucleotide (DNA and/or RNA) or an artificial nucleic acid may be present at one or both ends of shRNA of a base sequence in a single-stranded nucleic acid fragment. The number of nucleotides or the like at one or both ends of shRNA is not particularly limited. If such end is to be ligated to the aforementioned hairpin-shaped DNA, such number is preferably between 1 and 20.

The target gene of shRNA is not particularly limited. Accordingly, the nucleic acid molecule of the present invention can include shRNA for any target gene, in principle. When shRNA contains mature double-stranded miRNA, shRNA of a target gene of interest can be contained without particular limitation, provided that miRNA of a target gene is present in the genome.

The nucleic acid molecule of the present invention comprising, as a constitutive element, a double-stranded nucleic acid fragment including such siRNA base sequence is capable of silencing the target gene expression *in vivo* or in cells with greater stability than conventional siRNA.

### <Single-stranded miRNA precursor>

As described in the "Mature double-stranded miRNA" section above, the term "single-stranded miRNA precursor" refers to miRNA in the state of a single-stranded precursor before it is converted into mature single-stranded miRNA upon transcription from the genome and processing in the nucleus. Specific examples include pri-miRNA and pre-miRNA.

A sequence of a single-stranded miRNA precursor within a single-stranded nucleic acid fragment can be the same base sequence as that of wild-type miRNA encoded in the genome.

At least one nucleotide (DNA and/or RNA) or an artificial nucleic acid may be present at one or both ends of a single-stranded miRNA precursor in a single-stranded nucleic acid fragment. The number of nucleotides or the like at one or both ends of the single-stranded miRNA precursor is not particularly limited. If such end is to be ligated to the aforementioned hairpin-shaped DNA, such number is preferably between 1 and 20.

The target gene of a single-stranded miRNA precursor is not particularly limited. With the use of the nucleic acid molecule of the present invention, accordingly, miRNA for a gene to be regulated the expression can be used when it is present in the genome.

The nucleic acid molecule of the present invention comprising a single-stranded nucleic acid fragment including such single-stranded miRNA precursor may be used in an adequate manner, so that the expression of a target gene can be silenced specifically and with greater stability than is possible with existing miRNA.

### <Nucleic acid aptamer>

The term "nucleic acid aptamer" refers to a nucleic acid that binds to a target molecule via its conformation, and thereby inhibits or suppresses functions of such molecule. RNA aptamers constituted by RNA and DNA aptamers constituted by DNA are known. A nucleic acid aptamer contained in the nucleic acid of the present invention may be constituted by DNA, RNA, or a combination thereof. An RNA aptamer constituted by RNA is preferable because RNA has flexibility that enables the formation of more conformations compared with DNA, in general.

A nucleic acid aptamer generally has higher specificity and affinity to a target molecule than an antibody. Accordingly, a nucleic acid aptamer can specifically, directly, and firmly bind to a target molecule. Since the number of target amino acid residues necessary for binding may be smaller than that of an antibody, for example, a nucleic acid aptamer is superior to an antibody, when selective suppression of functions of a given protein among highly homologous proteins is intended. Regarding aptamers, reference can be made to Jaynasena S. D., 1999, Clin. Chem. 45: 1628-1650, for example.

At least one nucleotide (DNA and/or RNA) or a nucleic acid analogue may be present at one or both ends of a nucleic acid aptamer in a base sequence of a single-stranded nucleic acid fragment. The number of nucleotides or the like at one or both ends of the nucleic acid aptamer is not particularly limited. If such an end is to be ligated to the aforementioned hairpin-shaped DNA, such number is preferably between 1 and 30.

The target molecule of the nucleic acid aptamer is not particularly limited. Accordingly, the nucleic acid of the present invention can contain a nucleic acid aptamer for any target molecule, in principle.

A nucleic acid aptamer sequence may be designed based on a sequence of a nucleic acid aptamer obtained by a method known in the art or a known nucleic acid aptamer sequence. When a nucleic acid aptamer is to be prepared, an RNA aptamer is prepared via *in vitro* selection making use of the systematic evolution of Ligands by exponential enrichment (SELEX) method, for example. The SELEX method comprises selecting an RNA molecule bound to a target molecule from an RNA pool composed of RNA molecules each having random sequence regions and primer-binding regions at both ends thereof, amplifying the recovered RNA molecule via RT-PCR, performing transcription using the obtained cDNA molecule as a template, and using the resultant as an RNA pool for the subsequent procedure. Such procedure is repeated several times to several tens of times to select RNA with a stronger ability to bind to a target molecule. The base sequence lengths of the random sequence region and the primer binding region are not particularly limited. In general, the random sequence region comprises 20 to 100 bases and the primer binding region comprises 15 to 40 bases. Specificity to a target molecule may be enhanced by prospectively mixing molecules similar to the target molecule with RNA pools and using a pool consisting of RNA molecules that did not bind to the molecule of interest. An RNA molecule that was obtained as a final product by such technique is used as an RNA aptamer. The SELEX method is a known technique, and a specific method may be implemented in accordance with, for example, Pan et al. (Proc. Natl. Acad. Sci. U.S.A., 1995, 92: 11509-11513).

The nucleic acid of the present invention comprising a single-stranded nucleic acid fragment that includes such nucleic acid aptamer may be used in accordance with an adequate method, so that functions of a target molecule can be inhibited or suppressed specifically and with greater stability than is possible with existing nucleic acid aptamers.

### <Ribozyme>

The term "ribozyme" is a generic name for RNA that functions as a catalyst. The ribozyme is known, for example, to have catalytic functions of specifically cleaving a specific RNA site, to have catalytic functions of ligating nucleic acids, or to have catalytic functions for aminoacylation. The ribozyme of the present invention includes a deoxyribozyme composed of DNA, in addition to a ribozyme composed of RNA. Accordingly, the "ribozyme" of the present invention generally encompasses those constituted in the form of single-stranded RNA and/or DNA.

At least one nucleotide (DNA and/or RNA) or a nucleic acid analogue may be present at one or both ends of a ribozyme in the base sequence of a single-stranded nucleic acid fragment. The number of nucleotides or the like at one or both ends of the ribozyme is not particularly limited. If such end is to be ligated to the aforementioned hairpin-shaped DNA, such number is preferably between 1 and 20.

The target molecule of the ribozyme is not particularly limited. Accordingly, the nucleic acid of the present invention can contain a ribozyme for any target molecule, in principle.

### <Molecular beacon>

A "molecular beacon" is a hairpin-shaped single-stranded nucleic acid having a stem structure and a loop structure, and it is a tool for genetic analysis used as a probe for confirming the existence of a sequence complementary to a loop moiety. It is generally quenched because of the short distance between a fluorophore and a quencher. If a loop moiety contains a complementary sequence, however, the loop moiety hybridizes to the complementary sequence. This opens the hairpin structure, the fluorophore is separated from the quencher, and fluorescence is thus detected.

At least one nucleotide (DNA and/or RNA) or a nucleic acid analogue may be present at one or both ends of a molecular beacon in the base sequence of a single-stranded nucleic acid fragment. The number of nucleotides or the like at one or both ends of the molecular beacon is not particularly limited. If such end is to be ligated to the aforementioned hairpin-shaped DNA, such number is preferably between 1 and 20.

The target molecule of the molecular beacon is not particularly limited. Accordingly, the nucleic acid of the present invention can include a molecular beacon for any target molecule, in principle.

### <Riboswitch>

A "riboswitch" is a cis-element existing in a non-translational region at the 5' end of mRNA or the like, and it functions as a metabolite-sensitive gene switch. The riboswitch directly binds to a low-molecular-weight organic compound or the like to alter the mRNA conformation and regulates the gene expression.

At least one nucleotide (DNA and/or RNA) or a nucleic acid analogue may be present at one or both ends of the riboswitch in the base sequence of a single-stranded nucleic acid fragment. The number of nucleotides or the like at one or both ends of the riboswitch is not particularly limited. If such end is to be ligated to the aforementioned hairpin-shaped DNA, such number is preferably between 1 and 20.

The target molecule of the riboswitch is not particularly limited. Accordingly, the nucleic acid of the present invention can contain a riboswitch for any target molecules, in principle.

<U1 adaptor>

A "U1 adaptor" is a bifunctional single-stranded nucleic acid consisting of about 25 bases, and it comprises a 5'-"target domain" complementary to the 3'-end exon in the mRNA precursor of the target gene and a 3'-"U1 domain" having a sequence complementary to the 5' region of U1 snRNA (Goraczniak R. et al., 2009, Nat. Biotechnol., 27: 257-263). Upon introduction of the U1 adaptor into an organism, U1 snRNP containing U1 snRNA binds to a region in the vicinity of a poly A signal of the mRNA precursor of the target gene, and polyadenylation of such mRNA is specifically inhibited. As a result, the mRNA precursor of the target gene is unstabilized and then degraded in the nucleus. Thus, gene silencing takes place.

At least one nucleotide (DNA and/or RNA) or a nucleic acid analogue may be present at one or both ends of the U1 adaptor in the base sequence of a single-stranded nucleic acid fragment. The number of nucleotides or the like at one or both ends of the U1 adaptor is not particularly limited. If such an end is to be ligated to the aforementioned hairpin-shaped DNA, such number is preferably between 1 and 20.

The target gene of the U1 adaptor is not particularly limited. Accordingly, the nucleic acid of the present invention can contain a U1 adaptor for any target gene, in principle.

The nucleic acid of the present invention containing a single-stranded nucleic acid fragment including such U1 adaptor may be used in an adequate manner, so that functions of a target molecule can be inhibited or suppressed specifically and with greater stability than is possible with existing U1 adaptors.

### <Target molecule-binding nucleic acid fragment>

The target molecule-binding region is as described in the "Target molecule-binding nucleic acid fragment" section of "1-2-1. Double-stranded nucleic acid fragment" above. An example of a target molecule-binding nucleic acid fragment is decoy RNA that has an RNA binding region of a given selective splicing regulator (e.g., a donor site or acceptor site) or an RNA binding region of given miRNA (i.e., an miRNA-binding region of the target gene) and inhibits or suppresses functions of the given selective splicing regulator or miRNA.

### 2. Constitutional element of the nucleic acid molecule

### 2-1. Nucleic acid molecule in which a hairpin-shaped DNA is ligated to a double-stranded nucleic acid fragment

In a nucleic acid molecule in which the hairpin-shaped DNA is ligated to a double-stranded nucleic acid fragment, the hairpin-shaped DNA can be ligated to up to 4 ends; i.e., the 5' ends and the 3' ends of the double-stranded nucleic acid fragments. In the nucleic acid molecule of the present invention, the hairpin-shaped DNA may be ligated to 3' end of at least one of the double-stranded nucleic acid fragment, so as to exert the effects of the nucleic acid molecule of the present invention.

Embodiments of the ligation of the double-stranded nucleic acid fragment to a hairpin-shaped DNA are determined depending on the number of the hairpin-shaped DNAs subjected to ligation and types of functional nucleic acids contained in the double-stranded nucleic acid fragment.

### (1) When a nucleic acid has one hairpin-shaped DNA

There are two embodiments of the ligation of the double-stranded nucleic acid fragment to a hairpin-shaped DNA, as shown in Figure 3. In either case, at least one of two 3' terminal nucleotides from at least one 3' end of the nucleic acid fragment to which the hairpin-shaped DNA is ligated is modified as mentioned above. In such a case, any types of functional nucleic acids may be contained in the double-stranded nucleic acid fragment. Specifically, a base sequence of siRNA composed of RNA or of mature double-stranded miRNA may be contained. Alternatively, a base sequence of a target molecule-binding region such as decoy DNA composed of DNA may be contained.

### (I) Nucleic acid molecule in which the 5' end of the hairpin-shaped DNA is ligated to the 3' end of one nucleic acid fragment constituting a double-stranded nucleic acid fragment (Figure 3(A))

According to this embodiment, the 3' end of the hairpin-shaped DNA is not ligated to the 5' end of the other nucleic acid fragment constituting a double-stranded nucleic acid fragment. The nucleic acid molecule of the present invention according to this embodiment is composed of two nucleic acid fragments (i.e., a nucleic acid fragment comprising a nucleic acid fragment of a double-stranded nucleic acid fragment ligated to the hairpin-shaped DNA and the other nucleic acid fragment of the double-stranded nucleic acid fragment).

When the double-stranded nucleic acid fragment contains an siRNA base sequence according to this embodiment, whether the 5' end of a hairpin-shaped DNA is to be ligated to the 3' end of a nucleic acid fragment comprising an siRNA sense strand or that of a nucleic acid fragment comprising an antisense strand is not particularly limited. A nucleic acid molecule comprising the hairpin-shaped DNA ligated to the 3' end of a nucleic acid fragment comprising an siRNA sense strand as shown in Figure 4 (A) or a nucleic acid molecule comprising the hairpin-shaped DNA ligated to the 3' end of a nucleic acid fragment comprising an siRNA antisense strand, as shown in Figure 4 (B), may be used. The nucleic acid molecule shown in Figure 4(A) is preferred, in which the 5' end of the hairpin-shaped DNA is ligated to the 3' end of the nucleic acid fragment comprising an siRNA sense strand. This is because the results of research by the present inventors showed that the modification of the 3' end of the nucleic acid fragment comprising a sense strand offered stronger stability and resistance to degradation by a nucleolytic enzyme as well as high inhibitory effects on gene expression.

### (II) Nucleic acid molecule in which the 5' end of a hairpin-shaped DNA is ligated to the 3' end of one of two nucleic acid fragments constituting a double-stranded nucleic acid fragment and the 3' end of the hairpin-shaped DNA is ligated to the 5' end of the other nucleic acid fragment (Figure 3 (B))

According to this embodiment, the 5' end and the 3' end of the hairpin-shaped DNA are respectively ligated to two nucleic acid fragments constituting a double-stranded nucleic acid fragment. As a result, the nucleic acid molecule of the present invention according to this embodiment is constituted by a nucleic acid fragment comprising the constituents below sequentially ligated from the 5' end toward the 3' end:
one nucleic acid fragment of the double-stranded nucleic acid fragment;
the hairpin-shaped DNA; and
the other nucleic acid fragment of the double-stranded nucleic acid fragment.

When the double-stranded nucleic acid fragment of this embodiment comprises an siRNA base sequence, either of two nucleic acid molecules (I) and (II) below may be used. The nucleic acid molecule (i) (Figure 4(C)) is preferred, in which the 5' end of hairpin-shaped DNA is ligated to the 3' end of a nucleic acid fragment comprising a sense strand as mentioned above.

(i) the nucleic acid molecule comprising the constituents below sequentially ligated from the 5' end toward the 3' end, as shown in Figure 4 (C):
   a nucleic acid fragment comprising an siRNA sense strand;
   a hairpin-shaped DNA; and
   a nucleic acid fragment comprising an siRNA antisense strand; or
(ii) the nucleic acid molecule comprising the constituents below sequentially ligated from the 5' end toward the 3' end, as shown in Figure 4 (D):
   a nucleic acid fragment comprising an siRNA antisense strand;
   a hairpin-shaped DNA; and
   a nucleic acid fragment comprising an siRNA sense strand.

### (2) When a nucleic acid has two hairpin-shaped DNAs

Nucleic acids can be roughly classified into the following three embodiments represented by (I) to (III) depending on the number of non-ligated regions between the double-stranded nucleic acid fragment and a hairpin-shaped DNA. The nucleic acid of the present invention may be in accordance with any embodiment. In any of these cases, the hairpin-shaped DNA is ligated to the 3' end of at least one of two nucleic acid fragments, and at least one of two 3' terminal nucleotides of at least one nucleic acid fragment is modified as mentioned above. According to a preferred embodiment, the hairpin-shaped DNA is ligated to the 3' end of nucleic acid fragment(s), wherein at least one of two 3' terminal nucleotides from the ligated 3' end is modified. When the hairpin-shaped DNA is ligated to the 3' ends of both two nucleic acid fragments, at least one of two 3' terminal nucleotides of any one or both of the nucleic acid fragments may be modified. Any types of functional nucleic acids may be contained in the double-stranded nucleic acid fragment. Specifically, a base sequence of siRNA mainly constituted by RNA or mature double-stranded miRNA may be contained therein. Alternatively, a base sequence of a target molecule-binding region, such as decoy DNA, mainly constituted by DNA may be contained therein.

### (I) When a nucleic acid contains one non-ligated region

According to this embodiment, one non-ligated region exists between an end of one nucleic acid fragment constituting a double-stranded nucleic acid fragment and an end of a hairpin-shaped DNA. The nucleic acid molecule according to this embodiment is accordingly constituted in the form of a nucleic acid fragment comprising two nucleic acid fragments each constituting a double-stranded nucleic acid fragment ligated to one of two the hairpin-shaped DNAs, as shown in Figure 5. The nucleic acid molecule according to this embodiment can further be classified into the following two embodiments (i) and (ii) based on the position of a non-ligated region between the double-stranded nucleic acid fragment and the hairpin-shaped DNA.

(i) A nucleic acid comprising nucleic acid fragments and hairpin-shaped DNAs ligated from the 5' end toward the 3' end in the following order (Figure 5 (A)):
   one nucleic acid fragment constituting a double-stranded nucleic acid fragment;
   the hairpin-shaped DNA;
   the other nucleic acid fragment constituting a double-stranded nucleic acid fragment; and
   the hairpin-shaped DNA.

According to this embodiment, the 5' end of one nucleic acid fragment constituting a double-stranded nucleic acid fragment is not ligated to the 3' end of a hairpin-shaped DNA. In other words, the hairpin-shaped DNA is ligated to each of the 3' ends of the double-stranded nucleic acid fragment. Hence, of two 3'terminal nucleotides of each nucleic acid fragment, i.e., a total of 4 nucleotides, at least one up to 4 are modified as mentioned above.

When the double-stranded nucleic acid fragment contains an siRNA base sequence according to this embodiment, either a nucleic acid fragment comprising an siRNA sense strand or a nucleic acid fragment comprising an siRNA antisense strand may correspond to "one nucleic acid fragment" mentioned above without any particular limitation. Specifically, "one nucleic acid fragment" mentioned above may be a nucleic acid fragment comprising an siRNA sense strand, while "the other nucleic acid fragment" may be a nucleic acid fragment comprising an siRNA antisense strand, as shown in Figure 6 (A). Alternatively, "one nucleic acid fragment" mentioned above may be a nucleic acid fragment comprising an siRNA antisense strand and "the other nucleic acid fragment" may be a nucleic acid fragment comprising an siRNA sense strand, as shown in Figure 6 (C). The nucleic acid molecule as shown in Figure 6(A) or 6(C) is preferred, in which the hairpin-shaped DNA is ligated to the 3' end of the nucleic acid fragment comprising an siRNA sense strand.

(ii) A nucleic acid molecule comprising nucleic acid fragments and hairpin-shaped DNAs ligated from the 5' end toward the 3' end in the following order (Figure 5(B)):
   a hairpin-shaped DNA;
   one nucleic acid fragment constituting a double-stranded nucleic acid fragment;
   a hairpin-shaped DNA; and
   the other nucleic acid fragment constituting a double-stranded nucleic acid fragment.

According to this embodiment, the 3' end of a nucleic acid fragment constituting a double-stranded nucleic acid fragment is not ligated to the 5' end of hairpin-shaped DNA. When the double-stranded nucleic acid fragment contains an siRNA base sequence according to this embodiment, accordingly, either a nucleic acid fragment comprising an siRNA sense strand or a nucleic acid fragment comprising an siRNA antisense strand may correspond to "one nucleic acid fragment" mentioned above without any particular limitation, as with the case of (i) above. Specifically, "one nucleic acid fragment" mentioned above may be a nucleic acid fragment comprising an siRNA sense strand, and "the other nucleic acid fragment" may be a nucleic acid fragment comprising an siRNA antisense strand, as shown in Figure 6 (D). Alternatively, "one nucleic acid fragment" mentioned above may be a nucleic acid fragment comprising an siRNA antisense strand and "the other nucleic acid fragment" may be a nucleic acid fragment comprising an siRNA sense strand, as shown in Figure 6 (B).

### (II) When a nucleic acid contains two non-ligated regions

According to this embodiment, non-ligated regions exist between an end of each of the two nucleic acid fragments constituting a double-stranded nucleic acid fragment and an end of a hairpin-shaped DNA. The nucleic acid molecule according to this embodiment can further be classified into the embodiments (i) and (ii) below based on positions of non-ligated regions between the double-stranded nucleic acid fragment and the hairpin-shaped DNA, as shown in Figure 7(A) or (B).

### (i) A nucleic acid molecule comprising hairpin-shaped DNAs ligated to the 5' end and the 3' end of one nucleic acid fragment constituting a double-stranded nucleic acid fragment (Figure 7 (A))

The nucleic acid molecule of this embodiment is composed of two nucleic acid fragments; i.e., a nucleic acid fragment comprising two hairpin-shaped DNAs ligated to one nucleic acid fragment of a double-stranded nucleic acid fragment and the other nucleic acid fragment of the double-stranded nucleic acid fragment. In the nucleic acid molecule of this embodiment, specifically, a hairpin-shaped DNA is not ligated to either end (i.e., the 5' end or the 3' end) of the other nucleic acid fragment. Thus, two non-ligated regions exist between both ends of the other nucleic acid fragment and an end of each of two hairpin-shaped DNAs.

According to this embodiment, only the 3' end of one nucleic acid fragment is ligated to the 5' end of a hairpin-shaped DNA. Hence, of two 3' terminal nucleotides of this one nucleic acid fragment, at least one up to two are modified as mentioned above.

When the double-stranded nucleic acid fragment contains an siRNA base sequence according to this embodiment, "the other nucleic acid fragment" may be either a nucleic acid fragment comprising an siRNA sense strand or a nucleic acid fragment comprising an siRNA antisense strand. Specifically, "one nucleic acid fragment" mentioned above may be a nucleic acid fragment comprising an siRNA sense strand, and "the other nucleic acid fragment" may be a nucleic acid fragment comprising an siRNA antisense strand, as shown in Figure 8 (A).

### (ii) A nucleic acid molecule in which a hairpin-shaped DNA is ligated to 3' end of each of the nucleic acid fragments constituting a double-stranded nucleic acid fragment (Figure 7 (B))

The nucleic acid molecule according to this embodiment is composed of two nucleic acid fragments in which a hairpin-shaped DNA is ligated to 3' end of each of the nucleic acid fragments constituting a double-stranded nucleic acid fragment. In contrast to this, a hairpin-shaped DNA is not ligated to the 5' ends of both the nucleic acid fragments. Hence, two non-ligated regions exist between the 5' end of each of the two nucleic acid fragments constituting a double-stranded nucleic acid fragment and the 3' end of each hairpin-shaped DNA. In addition, at least one of two 3' terminal nucleotides of each nucleic acid fragment is modified. Specifically, of 4 nucleotides, at least one up to 4 are modified as mentioned above.

When a double-stranded nucleic acid fragment comprises an siRNA base sequence according to this embodiment of ligation, a nucleic acid comprise a hairpin-shaped DNA ligated to the 3' end of each of a nucleic acid fragment comprising a base sequence of the siRNA sense and antisense strands as shown in Figure 8 (B).

### (III) When a nucleic acid does not comprise a non-ligated region (Figure 9)

In the nucleic acid of this embodiment, all ends of the double-stranded nucleic acid fragment are ligated to the ends of two hairpin-shaped DNAs. Specifically, the nucleic acid of this embodiment comprises the following nucleic acid fragments and hairpin-shaped DNAs sequentially ligated from the 5' end toward the 3' end, and the 5' end of the nucleic acid fragments is ligated to the 3' end of hairpin-shaped DNAs:

one nucleic acid fragment constituting a double-stranded nucleic acid fragment;
the hairpin-shaped DNA;
the other nucleic acid fragment constituting a double-stranded nucleic acid fragment; and
the hairpin-shaped DNA.

According to this embodiment, a hairpin-shaped DNA is ligated to both the 3' ends of the double-stranded nucleic acid fragment without exception. Hence, of two 3' terminal nucleotides of each nucleic acid fragment, i.e., a total of 4 nucleotides, at least one up to 4 are modified as mentioned above.

The nucleic acid according to this embodiment is further classified into the following three embodiments depending on the positions and number of nicks in the double-stranded nucleic acid fragment region.

### (i) When a double-stranded nucleic acid fragment region comprises no nicks (Figure 9 (A))

According to this embodiment, the nucleic acid molecule of the present invention is in a closed circular state and forms a so-called dumbbell-shaped structure.

According to this embodiment, any types of functional nucleic acids may be contained in the double-stranded nucleic acid fragment. When the double-stranded nucleic acid fragment contains a base sequence of a target molecule-binding region mainly constituted by DNA, such as decoy DNA, such embodiment is particularly preferable.

### (ii) When any one of the nucleic acid fragment regions constituting a double-stranded nucleic acid fragment comprises a nick (Figure 9 (B))

According to this embodiment, the nucleic acid molecule of the present invention has a constitution similar to that of the nucleic acid described in "(2) When a nucleic acid has two hairpin-shaped DNAs, (I) When 2 nucleic acid contains one non-ligated region" above. It should be noted that the nucleic acid of this embodiment differs from the nucleic acid of (2) (I) above in that a nick (i.e., a non-ligated region) is located within a double-stranded nucleic acid fragment instead of a site between a nucleic acid fragment constituting a double-stranded nucleic acid fragment and a hairpin-shaped DNA.

According to this embodiment, an example of a functional nucleic acid contained in the double-stranded nucleic acid fragment is a target molecule-binding region mainly composed of DNA, such as decoy DNA. The nick may be located within the binding region.

### (iii) When each of the nucleic acid fragment regions constituting a double-stranded nucleic acid fragment has a nick (i.e., two nicks in total) and such nicks are not paired (Figure 9 (C)).

According to this embodiment, the nucleic acid molecule of the present invention has a constitution similar to that of the nucleic acid according to (2) (II) (ii) above. It should be noted that the nucleic acid molecule of this embodiment differs from the nucleic acid described in "(2) When a nucleic acid contains two hairpin-shaped DNAs (II) When a nucleic acid contains two non-ligated regions (ii)" above in that a nick (i.e., a non-ligated region) is located within a double-stranded nucleic acid fragment instead of a site between a nucleic acid fragment constituting a double-stranded nucleic acid fragment and a hairpin-shaped DNA.

The term "nicks are not paired" refers to a situation in which nicks are not present at the same corresponding sites in each of the nucleic acid fragment regions constituting a double-stranded nucleic acid fragment. In the nucleic acid of this embodiment, accordingly, at least 1 and preferably at least 2 base pairs are present between two nicks.

An example of a functional nucleic acid contained in the double-stranded nucleic acid fragment of this embodiment is a target molecule-binding region mainly composed of DNA, such as decoy DNA. The nick may be present within such binding region.

### 2-2. A nucleic acid molecule in which a hairpin-shaped DNA is ligated to a single-stranded nucleic acid fragment

A nucleic acid molecule in which a hairpin-shaped DNA is ligated to a single-stranded nucleic acid fragment can comprise the hairpin-shaped DNA ligated to the 3' end or the 5' end and/or the 3' end of the single-stranded nucleic acid fragment. At least one of two nucleotides from the 3' end has the above modification.

Embodiments of the ligation between the single-stranded nucleic acid fragment and a hairpin-shaped DNA are determined depending on the number of hairpin-shaped DNAs ligated.

### (1) When a nucleic acid has one hairpin-shaped DNA

As described below, there are two embodiments of the ligation between the single-stranded nucleic acid fragment and the hairpin-shaped DNA, as shown in Figure 10(A) and (B). The nucleic acid molecule of the present invention may be of any embodiment. Any types of functional nucleic acids may be contained in the single-stranded nucleic acid fragment. For example, a base sequence of a single-stranded miRNA precursor, shRNA, a nucleic acid aptamer, a ribozyme (including deoxyribozyme), a molecular beacon, riboswitch, a U1 adaptor, or a target molecule-binding region can be contained.

### (I) A nucleic acid in which the 5' end of a hairpin-shaped DNA is ligated to the 3' end of a single-stranded nucleic acid fragment (Figure 10 (A))

The nucleic acid of this embodiment is composed of a nucleic acid fragment comprising a single-stranded nucleic acid fragment and hairpin-shaped DNA ligated thereto. The 5' end of the nucleic acid is derived from a single-stranded nucleic acid fragment and the 3' end thereof is derived from hairpin-shaped DNA.

### (III) A nucleic acid in which the 5' end and the 3' end of hairpin-shaped DNA are ligated to the 3' end and the 5' end of a single-stranded nucleic acid fragment, respectively (Figure 10 (B))

The nucleic acid of this embodiment is in the form of a closed circular nucleic acid in which a single-stranded nucleic acid fragment is ligated to a hairpin-shaped DNA.

### (2) When a nucleic acid has two hairpin-shaped DNAs

The nucleic acid of this embodiment comprises two hairpin-shaped DNAs ligated to the 5' end and the 3' end of a single-stranded nucleic acid fragment (Figure 11).

The nucleic acid of this embodiment is in the form of a nucleic acid fragment comprising a single-stranded nucleic acid fragment ligated to two hairpin-shaped DNAs in such a manner that the single-stranded nucleic acid fragment is sandwiched by two hairpin-shaped DNAs.

In the nucleic acid of this embodiment, any types of functional nucleic acids can be contained in the single-stranded nucleic acid fragment region. For example, a base sequence of any of a single-stranded miRNA precursor, shRNA, a nucleic acid aptamer, a ribozyme (including deoxyribozyme), a molecular beacon, riboswitch, or a U1 adaptor can be contained.

### 3. Production of the nucleic acid molecule of the present invention

A method for producing the nucleic acid molecule of the present invention, i.e., a nucleic acid molecule with enhanced resistance to degradation by a nucleolytic enzyme, will be described.

The method for producing the nucleic acid molecule of the present invention comprises a modification step and a ligation step.

The "modification step" is the step of modifying a nucleotide at a given site in a nucleic acid fragment to be imparted with enhanced resistance to degradation by a nucleolytic enzyme (hereinafter, referred to as a "nucleic acid fragment to be resistant to degradation"). In this context, when the nucleic acid fragment to be resistant to degradation is a double-stranded nucleic acid fragment made by complete or partial base pairing, the given site specifically corresponds to the position of at least one of two 3'terminal nucleotides from at least one of two 3' ends. Alternatively, when the nucleic acid fragment to be resistant to degradation is a single-stranded nucleic acid fragment having at least one stem structure and at least one loop structure, the given site corresponds to the position of at least one of two 3' terminal nucleotides from the 3' end.

The "ligation step" is the step of ligating a hairpin-shaped DNA having the structure mentioned above to at least one 3' end of the nucleic acid fragment to be resistant to degradation. When the nucleic acid fragment to be resistant to degradation is a double-stranded nucleic acid fragment, the hairpin-shaped DNA may be ligated to one or both of two 3' ends existing in the nucleic acid fragment. Preferably, the hairpin-shaped DNA is ligated to at least one 3' end of the nucleic acid fragment(s) whose at least one of two 3' terminal nucleotides is modified in the modification step. Specifically, when any one nucleic acid fragment is modified, the hairpin-shaped DNA is preferably ligated to the 3' end of this nucleic acid fragment. When both the nucleic acid fragments are modified, the hairpin-shaped DNA is preferably ligated to at least one of the 3' ends of these two nucleic acid fragments.

The modification step and the ligation step may be performed in any order. Generally, the ligation step is achieved by: designing a nucleic acid molecule at a preliminary design stage such that a hairpin-shaped DNA is ligated to given 3' end(s) of the nucleic acid fragment to be resistant to degradation; and chemically synthesizing the nucleic acid molecule in accordance with a known solid-phase synthesis technique based on the sequence of the designed nucleic acid molecule. Regarding a chemical synthesis technique for nucleic acids, reference can be made to, for example, Current Protocols in Nucleic Acid Chemistry, Volume 1, Section 3. Also, this step may be achieved by independently preparing a nucleic acid fragment and a hairpin-shaped DNA and then ligating them using an enzyme such as DNA ligase. The modification step is achieved by: predetermining the position or number of modification before designing the sequence of the nucleic acid molecule of the present invention; and introducing the modification simultaneously with chemical synthesis. Many life sciences manufacturers (e.g., Takara Bio, Life Technologies Corporation, Sigma-Aldrich Corporation and GeneDesign Inc.) provide contract manufacturing services for chemical synthesis of the nucleic acid fragment in which a part of the nucleotide is modified, and services provided thereby can be utilized.

After chemical synthesis, the nucleic acid molecule of the present invention is preferably purified before use by a method known in the art. Examples of purification techniques include gel purification, affinity column purification, and HPLC.

When the nucleic acid molecule of the present invention is composed of two nucleic acid fragments (for example, when it has a structure shown in Figure 3 (A), Figure 7 (A) and Figure 7 (B), or Figure 9 (C)), the nucleic acid molecule of the present invention can be prepared by chemically synthesizing nucleic acid fragments independently, purifying the same as necessary, and mixing two nucleic acid fragments of preferably equivalent amounts so that they anneal with each other.

When the nucleic acid molecule of the present invention is composed of a nucleic acid fragment (for example, when it has a structure shown in Figure 3 (C), Figure 5 (A) and Figure 5 (B), Figure 9 (B), Figure 10 (A), or Figure 11), the nucleic acid molecule of the present invention can be prepared by chemically synthesizing a nucleic acid fragment, purifying the same as necessary, and placing the resultant under conditions that allow intramolecular folding.

When the nucleic acid molecule of the present invention has a closed-circular form as shown in Figure 9 (A) and Figure 10 (B), for example, a nucleic acid fragment having a nick at an adequate site within the nucleic acid is chemically synthesized, the resultant is purified as necessary, and both ends of the single nucleic acid fragment may be ligated to each other by a method known in the art. For example, such ends can be ligated biochemically with the use of an enzyme such as a ligase.

### 4. Effects

The nucleic acid molecule of the present invention can enhance resistance of a double-stranded nucleic acid fragment or a single-stranded nucleic acid fragment forming a higher-order structure via intramolecular folding to degradation by a nucleolytic enzyme, compared with the resistance attained when such nucleic acid fragment is used at least alone or when a known linker nucleic acid or a nucleic acid such as a hairpin-shaped nucleic acid that imparts degradation resistance is ligated to such nucleic acid fragment. Thus, the stability of the functional nucleic acids contained in the double-stranded nucleic acid fragments or the like *in* vivo can be enhanced, and pharmacological effects of such functional nucleic acids can be maintained and/or enhanced.

According to the present invention, in addition, the nucleic acid molecule of the present invention is capable of imparting resistance to degradation by a nucleolytic enzyme even it does not have a closed-circular form, such as a known dumbbell-shaped nucleic acid (i.e., even it comprises one or two non-ligated regions). The fact that the nucleic acid molecule has such a non-ligated region is very useful in respect of simplification of the preparation of functional nucleic acids or reduction in production costs. Specifically, when preparing the nucleic acid of the present invention, for example, the process of nucleic acid cyclization, which had heretofore been conducted with a dumbbell-shaped nucleic acid, can be omitted, and a dumbbell-shaped nucleic acid that had been chemically synthesized as a single long linear nucleic acid can be divided into two linear nucleic acids and then chemically synthesized.

Further, preparation of the nucleic acid molecule of the present invention can be fundamentally completed via chemical synthesis alone, and performance of purification and annealing is sufficient as subsequent processing. It is thus excellent in that nucleic acids of interest can be mass-produced in a cost-effective manner.

According to the method of the present invention for producing a nucleic acid molecule with enhanced resistance to degradation by a nucleolytic enzyme, resistance to degradation by a nucleolytic enzyme can be imparted to a nucleic acid fragment that had previously been susceptible to degradation by a nucleolytic enzyme and unstable *in vivo.* When a nucleic acid fragment to be resistant to degradation comprises a functional nucleic acid, accordingly, this method can maintain and/or enhance the functions and activity thereof for a longer period of time. Effects of such functional nucleic acid can be consequently enhanced.

### II. Pharmaceutical composition

The second aspect of the present invention relates to a pharmaceutical composition.

### 1. Constitution of a pharmaceutical composition

The pharmaceutical composition of the present invention comprises, as an active ingredient, the nucleic acid according to the first aspect.

The amount of the nucleic acid molecule of the present invention in the pharmaceutical composition of the present invention may be a pharmaceutically effective amount. The term "pharmaceutically effective amount" used herein refers to a dose that is necessary for an active ingredient of a pharmaceutical composition (i.e., a functional nucleic acid in the nucleic acid molecule of the present invention) to exert its functions that imposes no or substantially no side effects harmful to organisms to which the pharmaceutical composition is administered. The specific dose varies depending on the type of functional nucleic acid used, the target molecule, the dosage form to be employed, information regarding the subject, and the route of administration. When a pharmaceutical composition is administered to a human, a pharmaceutically effective amount and a preferable route of administration are generally determined based on the data obtained as a result of cell culture assays and animal experimentation. The final dose is determined and adjusted by a doctor in accordance with the individual subject. In such a case, examples of information regarding the subject that is to be taken into consideration include the extent or severity of a disease, general physical conditions, age, body weight, sexuality, eating habits, drug sensitivity, and resistance to treatment and the like.

A specific example of the amount of the nucleic acid molecule of the present invention per dose is about 0.01% (w/v) to about 20% (w/v), and preferably about 0.1% (w/v) to about 10% (w/v) when the nucleic acid molecule of the present invention containing siRNA is administered in the form of an injection to a human adult male (body weight: 60 kg) who is not in need of other pharmaceuticals. When administration of a large quantity of the nucleic acid molecule of the present invention is required in order to attain pharmacological effects of the pharmaceutical composition of the present invention, administration can be carried out in several separate instances in order to reduce burdens on a subject.

The pharmaceutical composition of the present invention can comprise a pharmaceutically acceptable carrier. The term "pharmaceutically acceptable carrier" refers to a solvent and/or additive that is generally used in the pharmaceutical field.

Examples of "solvents" include water (e.g., saline, buffer, and glucose solution) and pharmaceutically acceptable organic solvents (e.g., ethanol, propylene glycol, ethoxylated isostearyl alcohol, polyoxylated isostearyl alcohol, and polyoxyethylene sorbitan fatty acid esters). Such solvents are preferably sterilized. It is preferable that the solvents is adjusted to be isotonic with blood, as the saline, as necessary.

Examples of "additives" include excipients, adsorption inhibitors, binders, disintegrators, fillers, emulsifiers, flow modifiers, and lubricants.

Examples of excipients include saccharides, such as monosaccharides, disaccharides, cyclodextrins, and polysaccharides (specific examples include, but are not limited to, glucose, sucrose, lactose, raffinose, mannitol, sorbitol, inositol, dextrin, maltodextrin, starch, and cellulose), metal salts (e.g., sodium phosphate or calcium phosphate, calcium sulfate, and magnesium sulfate), citric acids, tartaric acids, glycine, low-, middle-, or high-molecular weight polyethylene glycol (PEG), Pluronic, and combinations of any thereof.

Examples of adsorption inhibitors include Tween 80, Tween 20, gelatin, and/or human serum albumin.

Examples of binders include starch pastes using maize, wheat, rice, or potato starch, gelatin, Tragacanth, methyl cellulose, hydroxypropyl methylcellulose, carboxymethylcellulose sodium, and/or polyvinyl pyrrolidone.

Examples of disintegrators include the aforementioned starch, carboxymethyl starch, crosslinked polyvinyl pyrrolidone, agar, alginic acid, sodium alginate, and salts of any thereof.

Examples of fillers include the aforementioned sugar and/or calcium phosphate (e.g., tricalcium phosphate and calcium hydrogen phosphate).

Examples of emulsifiers include sorbitan fatty acid ester, glycerine fatty acid ester, sucrose fatty acid ester, and propylene glycol fatty acid ester.

Examples of flow modifiers and lubricants include silicate, talc, stearate, and polyethylene glycol.

Such carriers are used to facilitate the preparation of pharmaceutical compositions and to maintain dosage forms and pharmacological effects thereof. Carriers may be adequately used as necessary. In addition to the aforementioned additives, stabilizers, flavoring agents, diluents, surfactants, solublizers, absorption promoters, humectants, extenders, moisturising agents, preservatives, antioxidants, buffers, or the like can be added, as necessary.

Further, the pharmaceutical composition of the present invention can comprise other drugs, provided that the pharmacological effects of the nucleic acid molecule of the present invention are maintained. For example, a given amount of antibiotics may be contained.

The dosage form of the pharmaceutical composition of the present invention is not particularly limited, provided that such dosage form does not inactivate a functional nucleic acid contained in the nucleic acid molecule of the present invention and allows the pharmacological effects thereof to be exerted *in vivo* after administration. For example, a liquid, solid, or semi-solid dosage form may be employed. Specific examples of dosage forms include parenteral dosage forms, such as injections, suspensions, emulsions, eye drops, nasal drops, creams, ointments, plasters, poultices, and suppositories, and oral dosage forms, such as liquid preparations, powders, granules, tablets, capsules, sublingual agents, and troches. In the present invention, the dosage form is preferably an injection since an active ingredient thereof is in the form of a nucleic acid.

### 2. Production of pharmaceutical composition

The pharmaceutical composition of the present invention may be produced by a method known in the art. For example, the method described in Remington's Pharmaceutical Sciences (Merck Publishing Co., Easton, Pa.) may be adopted.

### 3. Method of administration of pharmaceutical composition

The pharmaceutical composition is preferably administered in a dosage unit form. The pharmaceutical composition can be administered through an oral route, directly into the tissue (e.g., subcutaneous, intramuscular, or intravenous administration), or outside the tissue (e.g., percutaneous, instillation, nasal, or transrectal administration). The pharmaceutical composition of the present invention is preferably administered in a dosage form adequate for the method of administration. When the pharmaceutical composition is directly administered into tissue, for example, injection through the blood stream is preferable. Thus, the dosage form may be a liquid preparation (an injection solution).

When the pharmaceutical composition is administered in the form of an injection, the site of injection is not particularly limited, provided that the nucleic acid molecule of the present invention can exert its functions on the target molecule and achieve an objective of the administration of the pharmaceutical composition. Examples include intravenous, intraarterial, intrahepatic, intramuscular, intraarticular, intramedullary, intraspinal, intraventricular, percutaneous, subcutaneous, intracutaneous, intraperitoneal, intranasal, intestinal, and sublingual sites. An injection into a blood vessel, such as intraveneous or intraarterial injection, is preferable since this allows the pharmaceutical composition of the present invention to be immediately distributed throughout the body via the blood stream and invasiveness is relatively low. Alternatively, the pharmaceutical composition may be injected directly into a site at which pharmacological effects of the pharmaceutical composition of the present invention are needed, thus allowing a large amount of the pharmaceutical composition to directly act on the target site.

### Examples

### <Example 1: Preparation of nucleic acid molecule comprising siRNA>

The nucleic acid molecules of the present invention comprising the siRNA used for Example 2 were prepared.

### (Method)

Firefly luciferase mRNA was designated as the siRNA target to be used for the experiment, and various nucleic acid molecules were designed. The structures of various nucleic acid molecules and base sequences thereof are shown in Figures 12-1 to 12-3. In these figures, "Cont. 1" is control nucleic acids each consisting only of an unmodified double-stranded nucleic acid fragment comprising siRNA of firefly luciferase mRNA, which have a constitution similar to that of a conventional siRNA. Cont. 2 is an unmodified control nucleic acid molecule in which a hairpin-shaped DNA described herein is ligated to each of the 3' ends of the nucleic acid fragment comprising the sense strand and the nucleic acid fragment comprising the antisense strand of Cont. 1. Cont. 3 to 7 are control nucleic acid molecules having 2'OMe-modified nucleotides (in Figure 12-1, the bases are indicated in underlined boldface) at various sites in the nucleic acid fragment comprising the sense strand and the nucleic acid fragment comprising the antisense strand of Cont. 1. Cont. 8 is an unmodified control nucleic acid molecule in which a hairpin-shaped DNA described herein is ligated to both of the 5' end and the 3' end of the nucleic acid fragment comprising the sense strand. M1 to M31 are Cont. 2- or Cont. 8-based test nucleic acid molecules having 2'OMe-and/or F-containing nucleotides at various sites in the nucleic acid fragment comprising the sense strand and/or the nucleic acid fragment comprising the antisense strand of the double-stranded nucleic acid fragment containing siRNA against firefly luciferase mRNA. The 2'OMe-containing nucleotides are indicated in underlined boldface in the figures, and the F-containing nucleotides are indicated in italicized boldface in the figures.

Synthesis of nucleic acid fragments of the various nucleic acid molecules designed was entrusted to GeneDesign Inc. (Osaka, Japan), and synthesis was carried out in accordance with a known solid-phase synthesis technique to perform HPLC purification. Subsequently, the synthesized nucleic acid fragments were subjected to purification by gel electrophoresis, the equivalent amounts of base-paired nucleic acid fragments were mixed in a phosphate buffer (pH 7.4), and, the resulting mixture was heated at 75 °C and gradually cooled down to room temperature for annealing, and the nucleic acid molecule of the present invention was thus prepared.

### <Example 2: Inhibition of firefly luciferase gene expression by the nucleic acid molecule of the present invention in vitro>

In Example 2, inhibitory effects of various nucleic acid molecules containing siRNA prepared in Example 1 (i.e., Cont. 1 to Cont. 8 and M1 to M31) on firefly luciferase gene expression were examined. Inhibitory effects were assayed by introducing the various nucleic acids of Example 1 into HeLa cells or NIH3T3 cells via lipofection together with a plasmid that encodes the firefly luciferase gene as the target molecule and measuring the luciferase emission intensity to examine the inhibitory effects of the various nucleic acids on gene expression.

### (Method)

### (1) Cell culture

Cells were cultured under 5% CO₂ at 37°C and grown in each medium) containing 10% fetal bovine serum (FBS, JRH BIOSCIENCES). and antibiotics (final concentration; penicillin: 100 U/mL, and streptomycine: 100 µg/mL). The medium for the HeLa cells was a MEM medium (Eagle's Minimum Essential Medium, Sigma). The medium for the NIH3T3 cells was a DMEM medium (Dulbecco's Modified Eagle Medium, Sigma).

### (2) Introduction of various nucleic acids prepared in Example 1 and plasmids into culture cells

In the case of HeLa cells, cells were seeded on a 96-well plate at 1.5 x 10⁴ cells/well (100 µL) and cultured in an antibiotic-free MEM medium containing 10% fetal bovine serum for 24 hours. NIH3T3 cells were seeded on a 96-well plate at 2 x 10⁴ cells/well (100 µL) and cultured in an antibiotic-free DMEM medium containing 10% fetal bovine serum for 24 hours. Transfection was carried out with the use of 0.5 µL of Lipofectamine 2000 (Invitrogen) per well by adding 50 µL solution mixed with 200 ng of plasmids encoding the firefly luciferase gene (pGL3-control, Promega) per well, 200 ng of plasmids encoding the *Renilla reniformis* luciferase gene (pGL4.74 [hRluc/TK], Promega) per well, and various nucleic acids prepared in Example 1 to a final concentration of 0.1 nM in OPTI-MEM medium (Invitrogen). The IC₅₀ value was determined by carrying out seven 2-fold dilutions starting at 75 nM of various nucleic acids prepared in Example 1 above and adding 50 µL of solution mixed in OPTI-MEM medium (Invitrogen) to a final concentration to 0.5 nM.

### (3) Analysis of inhibitory effects on gene expression

Culture was continued for 22 hours after transfection, firefly luciferin emissions and *Renilla reniformis* luciferin emissions were quantified using the Dual-Luciferase Reporter Assay System (Promega), and inhibitory effects on protein expression of the firefly luciferase as an siRNA target were examined. Specifically, the cells were washed (twice for HeLa cells or once for NIH3T3 cells) with 100 µL of PBS per well after transfection, 20 µL of a lysis buffer was added thereto, and the cells were lysed with gentle agitation at 25°C for 30 minutes. The LARII reagent (100 µL) was added to and mixed with the solution, and firefly luciferin emissions were detected with the use of Multilabel Reader (PerkinElmer Japan). Subsequently, the Stop & Glo reagent (100 µL) was added to detect *Renilla reniformis* luciferase emissions, and each emission intensity was quantified with the Multilabel Reader (PerkinElmer Japan). When detecting emissions, as the background, the quantity of emissions from the samples without transfection was subtracted from that of each type of luciferin emission measured. The firefly luciferin emission indicating the expression level of firefly luciferase, which was a target molecule, was divided by the *Renilla reniformis* luciferin emissions indicating the expression level of *Renilla reniformis* luciferase, which was a control sample coexpressed therewith, and the assayed values were normalized. The value attained in the absence of an siRNA-containing nucleic acid was designated as 100%, and the relative activity of the firefly luciferase as the target molecule in the presence of a variety of siRNAs was determined. In addition, the relative activity of the luciferase as the target molecule in the presence of a variety of siRNAs with different concentrations was determined, and the IC₅₀ value was determined based on a graph plotting relative activity in relation to concentration. Specifically, the value of interest was determined with the use of KaleidaGraph (Albeck Software) by applying the obtained value to a calculation formula: Y = m4 + (m3 - m4) / (1 + 10^ ((LOG (m0) - LOG (m1))*m2)) [default value: m1 = m2 = m3 = 1, m4 = 100] carrying out data fitting using the least-square method. Y (%) is relative activity of luciferase as a target molecule in the presence of siRNA, m0 (nM) is the siRNA concentration, and the IC₅₀ value is m1 (nM).

### (Results)

The relative expression levels of the firefly luciferase gene in HeLa cells are shown in Figures 13-1 to 13-4, and the IC₅₀ values of inhibitory effects on its expression are shown in Table 1. The relative expression levels of the firefly luciferase gene in-NIH3T3 cells are shown in Figure 14.

**[Table 1]**

| NO. | siRNA | IC₅₀(pM) |
|---|---|---|
| 1 | Cont.1 | 32.6±9.7 |
| 2 | Cont.2 | 9.1±0.9 |
| 3 | M1 | 5.2±0.8 |
| 4 | M18 | 3.0±0.3 |
| 5 | M26 | 3.8±0.6 |

From the results of Figure 13-1, M1 to M3 were found to have higher inhibitory effects on expression than the nucleic acid molecule, represented by Cont. 1, consisting only of the conventional siRNA. M1 and M2 had higher inhibitory effects on expression than the nucleic acid molecule, represented by Cont. 2, in which the hairpin-shaped DNA was merely ligated to the 3' end of the siRNA sense strand (M3 was equivalent to Cont. 2). The results demonstrated that it is important to modify (here, 2'OMe modification) at least one of two 3' terminal nucleotides of the sense strand or the antisense strand. Particularly, when the nucleic acid fragment contains siRNA, the 3' terminal modification of the sense strand was found to be able to produce higher inhibitory effects on expression. These enhanced inhibitory effects on expression are considered to be ascribable to enhancement in the resistance of siRNA to degradation by a nucleolytic enzyme.

In contrast to this, when a large number of 2'OMe-modified nucleotides were introduced to various sites in one or both of the nucleic acid fragments constituting the double-stranded nucleic acid fragment (Cont. 4 to 7 and M4 to M14), most of the resulting nucleic acid molecules were found to rather reduce inhibitory effects on expression with respect to Cont. 1. These results demonstrated that not the number of modified nucleotides but the position of nucleotides to be modified, i.e., modification of at least one of two 3' terminal nucleotides of at least one nucleic acid fragment (preferably, a nucleic acid fragment with a hairpin-shaped DNA-ligated 3' end), is important for enhancement in the degradation resistance of the nucleic acid molecule by means of modification. When at least one of two 3' terminal nucleotides of the nucleic acid fragment comprising the sense strand was modified, more enhanced degradation resistance and high inhibitory effects on gene expression were also found to be obtained.

In Figure 13-2, examination was made on constitutionally Cont. 2-based nucleic acid molecules (M15 to M27) in which two 3' terminal nucleotides of at least one nucleic acid fragment were 2'OMe-modified and/or F-modified in various combinations. As a result, all the nucleic acid molecules had higher inhibitory effects on expression than Cont. 1 having the same constitution as the conventional siRNA. M18 to M20 and M23 to M27 had significantly higher inhibitory effects on expression than Cont. 2. These results demonstrated again that, as shown in Figure 13-1, it is important to modify at least one of two 3' terminal nucleotides from at least one hairpin-shaped DNA-ligated 3' end. When the 3' terminal nucleotide of the sense strand was F-modified as in M15 to M17, the resulting nucleic acid molecules exhibited higher inhibitory effects on expression than Cont. 1 without hairpin-shaped DNA. It was therefore confirmed that the modification is not necessarily required to be 2'OMe modification.

In Figure 13-3, Cont. 3 in which a hairpin-shaped DNA was not ligated and each 3' end of the siRNA-containing nucleic acid fragment was 2'OMe-modified, the nucleic acid molecule (M28) in which a hairpin-shaped DNA was ligated to both ends of only the sense strand and the 3' end of only the sense strand was 2'OMe-modified, and the nucleic acid molecule (M29) in which the 3' ends of the sense strand and the antisense strand were 2'OMe-modified were examined for their inhibitory effects on expression. As a result, Cont. 3 had inhibitory effects on expression almost equivalent to Cont. 1. This shows that mere modification of at least one of two 3'terminal nucleotides does not suffice for enhancement in the degradation resistance of the nucleic acid molecule, whereas this modification combined with hairpin DNA is important therefor.

Even the nucleic acid molecules (M28 and M29) in which a hairpin-shaped DNA was ligated to only one nucleic acid fragment (here, sense strand) were confirmed to have inhibitory effects on expression equivalent to or higher than Cont. 2. The results demonstrated that when the nucleic acid fragment is double-stranded (here, siRNA), enhancement in inhibitory effects on expression does not require that a hairpin-shaped DNA should be ligated to the 3' ends of both the nucleic acid fragments (here, sense strand and antisense strand), whereas ligation of a hairpin-shaped DNA to the 3' end of at least one nucleic acid fragment suffices.

In Figure 13-4, the unmodified nucleic acid molecule Cont. 8 in which a hairpin-shaped DNA was ligated to both ends of only the sense strand, the nucleic acid molecule (M28) in which the 3' end of only the sense strand was 2'OMe-modified, and the nucleic acid molecule (M30) in which, as in M29, the 3' ends of the sense strand and the antisense strand were 2'OMe-modified and the number of modified nucleotides at the 3' end of the antisense strand that was not ligated with a hairpin-shaped DNA was set to two were examined for their inhibitory effects on expression. The results about M31 demonstrated again that ligation of a hairpin-shaped DNA to the 3' end of at least one nucleic acid fragment suffices for enhancement in the degradation resistance of the nucleic acid molecule. The modification of two nucleotides, rather than one nucleotide, at the 3' end of the nucleic acid fragment that was not ligated with a hairpin-shaped DNA was found to further enhance the degradation resistance.

The results of Figure 14 demonstrated that the nucleic acid molecule of the present invention exhibits similar effects in various types of cells, because the same effects as in the HeLa cells were also obtained in the NIH3T3 cells.

### <Example 3: Examination of degradation resistance to mouse serum>

The nucleic acid molecule of the present invention was examined for its resistance to degradation by a nucleolytic enzyme.

### (Method)

The stability of the nucleic acid molecule of the present invention in which nucleotides at the hairpin-shaped DNA-ligated 3' ends of siRNA were 2'OMe-modified was examined in mouse serum containing various nucleolytic enzymes.

Cont. 1, Cont. 2, and the nucleic acid molecule M1 of the present invention were prepared in a buffer (1 mM KH₂PO4, 155 mM NaCl, 3 mM Na₂HPO₄, pH 7.4) to a final concentration to 1.25 µM, then heated at 75°C and gradually cooled down to room temperature for annealing. Mouse serum was then mixed therewith to a final concentration to 80%, and the resulting mixture was incubated at 37°C for 30 minutes (0.5 hours), 1 hour, 6 hours, and 22 hours. In this experiment, the start time of the incubation was adjusted such that the reactions of these samples were terminated at the same time. The nucleic acid degradation reaction was terminated by adding 30 µL of 500 mM EDTA (pH 8.0) solution and 50 µL of 10 M urea solution to 20 µL of reaction solution. The degradation product was separated by electrophoresis on 7 M urea-15% polyacrylamide gel. The band pattern was stained with SYBR Green II and analyzed with the use of a bio-imaging analyzer FLA7000 (Fuji Film).

### (Results)

The results are shown in Figure 15. A band corresponding to a full-length fragment of Cont. 1 containing no hairpin-shaped DNA almost disappeared after 30 minutes in the presence of serum and became totally undetectable after 6 hours. On the other hand, a full-length band was detectable even after 22 hours in the unmodified Cont. 2 in which a hairpin-shaped DNA was ligated to the 3' ends of siRNA, and the nucleic acid molecule M1 of the present invention in which a hairpin-shaped DNA was ligated to the 3' ends of siRNA and the nucleotides at these 3' ends were 2'OMe-modified. The nucleic acid fragments of the nucleic acid molecule M1 were less partially degraded than Cont. 2, as seen from the pattern of a band (indicated by arrowhead in the figure) corresponding to a partially degraded nucleic acid fragment, detected below the band corresponding to the full-length nucleic acid fragment. These results demonstrated that the nucleic acid molecule of the present invention is more stable in serum than Cont. 2 in which a hairpin-shaped DNA was merely ligated to the 3' ends. This suggests that the nucleic acid molecule of the present invention has high resistance to degradation by a nucleolytic enzyme.

All publications, patents, and patent applications cited herein are incorporated herein by reference in their entirety.

## Claims

1. A nucleic acid molecule comprising;
a hairpin-shaped DNA comprising nucleic acid regions (A) to (C) below sequentially ligated from the 5' end toward the 3' end:
(A) a first nucleic acid region consisting of 2 to 5 arbitrary nucleotides;
(B) a second nucleic acid region consisting of a "gna" or "gnna" base sequence, wherein each "n" independently represents "g", "t", "a", or "c", a base analogue, or a modified base; and
(C) a third nucleic acid region consisting of a base sequence complementary to the first nucleic acid region,
wherein the first nucleic acid region and the third nucleic acid region form a stem moiety by base pairing with each other and the second nucleic acid region forms a loop moiety, and
a nucleic acid fragment (1) or (2) below:
(1) a double-stranded nucleic acid fragment made by complete or partial base pairing, wherein at least one of two 3' terminal nucleotides from at least one 3' end is modified; or
(2) a single-stranded nucleic acid fragment having at least one stem structure and at least one loop structure, wherein at least one of two 3' terminal nucleotides is modified, wherein at least one 3' end of the nucleic acid fragment is ligated to the hairpin-shaped DNA.

2. The nucleic acid molecule according to claim 1, wherein the nucleic acid fragment is the double-stranded nucleic acid fragment (1) of claim 1, wherein at least one hairpin-shaped DNA-ligated nucleic acid strand of the nucleic acid fragment is modified.

3. The nucleic acid molecule according to claim 1 or 2, wherein the first nucleic acid region consists of "g" or "c" base.

4. The nucleic acid molecule according to any one of claims 1 to 3, wherein the modified nucleotide in the nucleic acid fragment is composed of RNA.

5. The nucleic acid molecule according to claim 4, wherein the modification is substitution of a 2'-hydroxy group in ribose.

6. The nucleic acid molecule according to claim 5, wherein the hydroxy group is substituted by a methoxy group, an ethoxy group, a propoxy group, or a butoxy group.

7. The nucleic acid molecule according to any one of claims 1 to 6, wherein the nucleic acid fragment comprises a functional nucleic acid.

8. The nucleic acid molecule according to claim 7, wherein the nucleic acid fragment is the double-stranded nucleic acid fragment (1) of claim 1, wherein the functional nucleic acid is an siRNA, a mature double-stranded miRNA, or a target molecule-binding nucleic acid fragment.

9. The nucleic acid molecule according to claim 7, wherein the nucleic acid fragment is the single-stranded nucleic acid fragment (2) of claim 1, wherein the functional nucleic acid is an shRNA, a single-stranded miRNA precursor, a nucleic acid aptamer, a ribozyme (including deoxyribozyme), a molecular beacon, a riboswitch, a U1 adaptor, or a target molecule-binding nucleic acid fragment.

10. A pharmaceutical composition comprising, as an active ingredient, the nucleic acid molecule according to any one of claims 1 to 9.

11. The pharmaceutical composition according to claim 10, which comprises a pharmaceutically acceptable carrier.

12. A method for producing a nucleic acid molecule with enhanced resistance to a nucleolytic enzyme, comprising:
a modification step of modifying
(1) at least one of two 3' terminal nucleotides from at least one 3' end of a double-stranded nucleic acid fragment made by complete or partial base pairing, or
(2) at least one of two 3' terminal nucleotides in a single-stranded nucleic acid fragment having at least one stem structure and at least one loop structure; and
a ligation step of ligating, to at least one 3' end of the nucleic acid fragment,
a hairpin-shaped DNA comprising the nucleic acid regions (A) to (C) below sequentially ligated from the 5' end toward the 3' end:
(A) a first nucleic acid region consisting of 2 to 5 arbitrary nucleotides;
(B) a second nucleic acid region consisting of a "gna" or "gnna" base sequence, wherein each "n" independently represents "g", "t", "a", or "c", a base analogue, or a modified base; and
(C) a third nucleic acid region consisting of a base sequence complementary to the first nucleic acid region,
wherein the first nucleic acid region and the third nucleic acid region form a stem moiety by base pairing with each other and the second nucleic acid region forms a loop moiety.

13. The production method according to claim 12, wherein the nucleic acid fragment is the double-stranded nucleic acid fragment (1) of claim 12, wherein the ligation step involves ligating the hairpin-shaped DNA to at least one modified nucleic acid strand of the nucleic acid fragment.

14. The production method according to claim 12 or 13, wherein the first nucleic acid region consists of "g" or "c" base.

15. The production method according to any one of claims 12 to 14, wherein the modified nucleotide in the nucleic acid fragment is composed of RNA.

16. The production method according to claim 15, wherein the modification is substitution of a 2'-hydroxy group in ribose.

17. The production method according to claim 16, wherein the hydroxy group is substituted by a methoxy group, an ethoxy group, a propoxy group, or a butoxy group.
